# EUROPEAN PATENT APPLICATION

(11) **EP 2 505 212 A2**
(43) Date of publication of application: **03.10.2012**
(21) Application number: 12158677.0
(22) Date of filing: 29.08.2007
(51) Int. Cl.: A61K 48/00

(54) **Novel P falciparum vaccine proteins and coding sequences**

(30) Priority: 29.08.2006 US 823897 P
(62) Divisional of application: 07811592.0
(71) Applicant: The United States of America as Represented By the Secretary of the Army, Walter Reed Army Institute of Research, Fort Detrick, MD 21702 (US); Kincaid, Randall L., Rockville MD 20850 (US)
(72) Inventor: Kincaid, Randall L., Rockville 20850 (US); Angov, Evelina, Silver Spring, MD Maryland 20910 (US); Lyon, Jeffrey A., Silver Spring, MD Maryland 20910 (US)
(74) Representative: Tombling, Adrian George

(57) **Abstract**

The present invention is directed to recombinant DNA technology and immunology, particularly antigens and related vaccines for prevention of malarial infection. The present invention includes novel recombinant malarial proteins, and fragments of the same, that have unique structural and/or chemical properties that allow them to be expressed in greater quantity in heterologous systems. These recombinant proteins and fragments have immunogenic properties superior to malarial proteins expressed using different expression systems and coding sequences, but having the same primary amino acid sequence.

SEQUENCE LISTING

<110> GOVERNMENT OF THE UNITED STATES OF AMERICA AS
      REPRESENTED BY THE SECRETARY OF THE ARMY
      KINCAID, RANDALL L.

<120> NOVEL P. FALCIPARUM VACCINE PROTEINS AND CODING
      SEQUENCES

<130> 38644-250467

<140>
<141>

<150> 60/823,897
<151> 2006-08-29

<160> 11

<170> PatentIn Ver. 3.3

<210> 1
<211> 1176
<212> DNA
<213> Artificial Sequence

<220>
<223> Description of Artificial Sequence: Synthetic
      construct

<400> 1
atggcacacc atcatcatca tcatcccggg ggatccggtt ctggtaccat ggctattagc 60
gtcactatgg acaatatatt aagcggcttc gaaaacgaat acgacgttat ctacctgaaa 120
ccactggctg gcgtttaccg ttcgctgaaa aaacagattg aaaaaaatat tttcaccttc 180
aacctgaacc tcaatgacat actgaacagc cgactcaaga aacgaaaata ctttctggac 240
gttctggaaa gcgacctgat gcagttcaaa cacatttcga gcaacgaata tattattgaa 300
gacagcttca aactgctcaa cagcgaacag aaaaataccc tcctgaaatc ttataaatac 360
atcaaagaaa gcgttgaaaa cgacattaaa ttcgctcaag aaggcatctc ttactacgaa 420
aaggttctgg ccaaatacaa ggacgacctg gaaagcatta aaaaagttat aaaagaagaa 480
aaggagaagt ttccaagcag cccaccaacc actccaccca gcccagctaa aaccgatgaa 540
cagaagaagg aaagcaagtt tctcccattc ctgaccaata ttgagacgct gtataacaat 600
ctggttaaca aaattgatga ctatctgatt aatctgaagg ctaagattaa tgactgcaac 660
gttgaaaaag acgaagctca cgttaaaatc actaaactct ctgacctgaa agctattgac 720
gataaaatcg acctcttcaa aaatccaacg gattttgaag ctattaaaaa actcatcaac 780
gacgacacga aaaaagacat gctcgggaaa ctgctctcta ccggcctggt tcagattttc 840
ccaaacacca tcatcagcaa actgattgaa ggcaaatttc aggacatgct gaatattagc 900
cagcatcagt gtgttaaaaa acagtgccca gaaaacagcg gctgctttcg tcacctggac 960
gaacgtgaag aatgcaaatg cctgctgaac tataaacagg aaggcgacaa atgcgttgaa 1020
aacccaaacc caacttgcaa tgaaaacaac ggcggctgcg acgctgacgc cacctgcacg 1080
gaagaagaca gcggctcgtc gcgtaagaaa ataacctgcg aatgcactaa accagacagc 1140
tacccactct ttgacggcat tttttgtagc taatga                           1176


<210> 2
<211> 390
<212> PRT
<213> Plasmodium falciparum

<400> 2
Met Ala His His His His His His Pro Gly Gly Ser Gly Ser Gly Thr
  1               5                  10                  15

Met Ala Ile Ser Val Thr Met Asp Asn Ile Leu Ser Gly Phe Glu Asn
             20                  25                  30

Glu Tyr Asp Val Ile Tyr Leu Lys Pro Leu Ala Gly Val Tyr Arg Ser
        35                  40                  45

Leu Lys Lys Gln Ile Glu Lys Asn Ile Phe Thr Phe Asn Leu Asn Leu
    50                  55                  60

Asn Asp Ile Leu Asn Ser Arg Leu Lys Lys Arg Lys Tyr Phe Leu Asp
65                  70                  75                  80

Val Leu Glu Ser Asp Leu Met Gln Phe Lys His Ile Ser Ser Asn Glu
                85                  90                  95

Tyr Ile Ile Glu Asp Ser Phe Lys Leu Leu Asn Ser Glu Gln Lys Asn
            100                 105                 110

Thr Leu Leu Lys Ser Tyr Lys Tyr Ile Lys Glu Ser Val Glu Asn Asp
        115                 120                 125

Ile Lys Phe Ala Gln Glu Gly Ile Ser Tyr Tyr Glu Lys Val Leu Ala
    130                 135                 140

Lys Tyr Lys Asp Asp Leu Glu Ser Ile Lys Lys Val Ile Lys Glu Glu
145                 150                 155                 160

Lys Glu Lys Phe Pro Ser Ser Pro Pro Thr Thr Pro Pro Ser Pro Ala
                165                 170                 175

Lys Thr Asp Glu Gln Lys Lys Glu Ser Lys Phe Leu Pro Phe Leu Thr
            180                 185                 190

Asn Ile Glu Thr Leu Tyr Asn Asn Leu Val Asn Lys Ile Asp Asp Tyr
        195                 200                 205

Leu Ile Asn Leu Lys Ala Lys Ile Asn Asp Cys Asn Val Glu Lys Asp
    210                 215                 220

Glu Ala His Val Lys Ile Thr Lys Leu Ser Asp Leu Lys Ala Ile Asp
225                 230                 235                 240

Asp Lys Ile Asp Leu Phe Lys Asn Pro Thr Asp Phe Glu Ala Ile Lys
                245                 250                 255

Lys Leu Ile Asn Asp Asp Thr Lys Lys Asp Met Leu Gly Lys Leu Leu
            260                 265                 270

Ser Thr Gly Leu Val Gln Ile Phe Pro Asn Thr Ile Ile Ser Lys Leu
        275                 280                 285

Ile Glu Gly Lys Phe Gln Asp Met Leu Asn Ile Ser Gln His Gln Cys
    290                 295                 300

Val Lys Lys Gln Cys Pro Glu Asn Ser Gly Cys Phe Arg His Leu Asp
305                 310                 315                 320

Glu Arg Glu Glu Cys Lys Cys Leu Leu Asn Tyr Lys Gln Glu Gly Asp
                325                 330                 335

Lys Cys Val Glu Asn Pro Asn Pro Thr Cys Asn Glu Asn Asn Gly Gly
            340                 345                 350

Cys Asp Ala Asp Ala Thr Cys Thr Glu Glu Asp Ser Gly Ser Ser Arg
        355                 360                 365

Lys Lys Ile Thr Cys Glu Cys Thr Lys Pro Asp Ser Tyr Pro Leu Phe

370                 375                 380

Asp Gly Ile Phe Cys Ser
385                 390



<210> 3
<211> 1056
<212> DNA
<213> Artificial Sequence

<220>
<223> Description of Artificial Sequence: Synthetic
      construct

<400> 3
   atggcacacc atcatcatca tcatcccggg ggatccggtt ctggtaccgc agtaactcct 60
   tccgtaattg ataacatact ttctaaaatt gaaaatgaat atgaggtttt atatttaaaa 120
   cctttagcag gtgtttatag aagtttaaaa aaacaattag aaaataacgt tatgacattt 180
   aatgttaatg ttaaggatat tttaaattca cgatttaata aacgtgaaaa tttcaaaaat 240
   gttttagaat cagatttaat tccatataaa gatttaacat caagtaatta tgttgtcaaa 300
   gatccatata aatttcttaa taaagaaaaa agagataaat tcttaagcag ttataattat 360
   attaaggatt caatagatac ggatataaat tttgcaaatg atgttcttgg atattataaa 420
   atattatccg agaaatataa atcagattta gattcaatta aaaaatatat aaacgacaaa 480
   caaggtgaaa atgagaaata ccttcccttt ttaaacaata ttgagacctt atataaaaca 540
   gttaatgata aaattgattt atttgtaatt catttagaag caaaagttct aaattataca 600
   tatgagaaat caaacgtaga agttaaaata aaagaactta attacttaaa aacaattcaa 660
   gacaaattgg cagattttaa aaaaaataac aatttcgttg gaattgctga tttatcaaca 720
   gattataacc ataataactt attgacaaag ttccttagta caggtatggt ttttgaaaat 780
   ctcgctaaaa ccgttttatc taatttactt gatggaaact tgcaaggtat gttaaacatt 840
   tcacaacacc aatgcgtaaa aaaacaatgt ccacaaaatt ctggatgttt cagacattta 900
   gatgaaagag aagaatgtaa atgtttatta aattacaaac aagaaggtga taaatgtgtt 960
   tgtaccgaag aagattcagg tagcaacgga aagaaaatca catgtgaatg tactaaacct 1020
   gattcttatc cacttttcga tggtattttc tgcagt                          1056



<210> 4
<211> 371
<212> PRT
<213> Plasmodium falciparum

<400> 4
Met Ala His His His His His His Pro Gly Gly Ser Gly Ser Gly Thr
1               5                   10                  15

Ala Val Thr Pro Ser Val Ile Asp Asn Ile Leu Ser Lys Ile Glu Asn
            20                  25                  30

Glu Tyr Glu Val Leu Tyr Leu Lys Pro Leu Ala Gly Val Tyr Arg Ser
        35                  40                  45

Leu Lys Lys Gln Leu Glu Asn Asn Val Met Thr Phe Asn Val Asn Val
    50                  55                  60

Lys Asp Ile Leu Asn Ser Arg Phe Asn Lys Arg Glu Asn Phe Lys Asn
65                  70                  75                  80

Val Leu Glu Ser Asp Leu Ile Pro Tyr Lys Asp Leu Thr Ser Ser Asn
                85                  90                  95

Tyr Val Val Lys Asp Pro Tyr Lys Phe Leu Asn Lys Glu Lys Arg Asp
            100                 105                 110

Lys Phe Leu Ser Ser Tyr Asn Tyr Ile Lys Asp Ser Ile Asp Thr Asp
        115                 120                 125

Ile Asn Phe Ala Asn Asp Val Leu Gly Tyr Tyr Lys Ile Leu Ser Glu
    130                 135                 140

Lys Tyr Lys Ser Asp Leu Asp Ser Ile Lys Lys Tyr Ile Asn Asp Lys
145                 150                 155                 160

Gln Gly Glu Asn Glu Lys Tyr Leu Pro Phe Leu Asn Asn Ile Glu Thr
                165                 170                 175

Leu Tyr Lys Thr Val Asn Asp Lys Ile Asp Leu Phe Val Ile His Leu
            180                 185                 190

Glu Ala Lys Val Leu Asn Tyr Thr Tyr Glu Lys Ser Asn Val Glu Val
        195                 200                 205

Lys Ile Lys Glu Leu Asn Tyr Leu Lys Thr Ile Gln Asp Lys Leu Ala
    210                 215                 220

Asp Phe Lys Lys Asn Asn Asn Phe Val Gly Ile Ala Asp Leu Ser Thr
225                 230                 235                 240

Asp Tyr Asn His Asn Asn Leu Leu Thr Lys Phe Leu Ser Thr Gly Met
                245                 250                 255

Val Phe Glu Asn Leu Ala Lys Thr Val Leu Ser Asn Leu Leu Asp Gly
            260                 265                 270

Asn Leu Gln Gly Met Leu Asn Ile Ser Gln His Gln Cys Val Lys Lys
        275                 280                 285

Gln Cys Pro Gln Asn Ser Gly Cys Phe Arg His Leu Asp Glu Arg Glu
    290                 295                 300

Glu Cys Lys Cys Leu Leu Asn Tyr Lys Gln Glu Gly Asp Lys Cys Val
305                 310                 315                 320

Glu Asn Pro Asn Pro Thr Cys Asn Glu Asn Asn Gly Gly Cys Asp Ala
                325                 330                 335

Asp Ala Lys Cys Thr Glu Glu Asp Ser Gly Ser Asn Gly Lys Lys Ile
            340                 345                 350

Thr Cys Glu Cys Thr Lys Pro Asp Ser Tyr Pro Leu Phe Asp Gly Ile
        355                 360                 365

Phe Cys Ser
    370

<210> 5
<211> 1119
<212> DNA
<213> Artificial Sequence

<220>
<223> Description of Artificial Sequence: Synthetic
      construct

<400> 5
atggcacacc atcatcatca tcatcccggg ggatccggtt ctggtaccgc tgttactcca 60
tcggttattg acaatatcct cagcaaaatt gaaaacgaat acgaggttct gtacctgaaa 120
ccactggctg gcgtttaccg ttctctgaaa aaacagctgg aaaacaatgt tatgaccttc 180
aacgttaacg ttaaggacat tctgaacagc cgattcaaca aacgagaaaa ctttaaaaac 240
gttctggaaa gcgacctgat tccatacaaa gacctgacca gctctaacta cgttgtcaaa 300

gacccataca aattcctcaa caaagaaaaa cgtgacaaat ttctgtcgtc ttacaactac 360
attaaggaca gcatcgacac ggacatcaac ttcgctaacg acgttctcgg ctactacaaa 420
atcctgtcgg aaaaatacaa aagcgacctg gacagcatta aaaaatacat aaatgataaa 480
cagggcgaaa acgagaaata tctccctttc ctgaataaca ttgagacgct gtacaaaacc 540
gttaacgaca aaattgacct gttcgttatt cacctggaag ctaaagttct caactacacc 600
tacgagaaaa gcaatgttga agttaaaatc aaagaactca actatctgaa aaccattcag 660
gataaactcg ctgacttcaa aaaaaacaat aactttgttg gcattgctga cctgagcacc 720
gactacaatc acaacaatct gctcaccaag tttctctcta ccggcatggt tttcgaaaac 780
ctcgctaaaa cggttctgag caacctgctc gacggcaatc tccagggcat gctgaatatt 840
agccagcatc agtgtgttaa aaaacagtgc ccacagaaca gcggctgctt tcgtcacctg 900
gacgaacgtg aagaatgcaa atgcctgctg aactataaac aggaaggcga caaatgcgtt 960
gaaaacccaa acccaacttg caatgaaaac aacggcggct gcgacgctga cgccaaatgc 1020
acggaagaag acagcggctc gaatggcaag aaaataacct gcgaatgcac taaaccagac 1080
agctacccac tctttgacgg cattttttgt tcttaatga 1119

<210> 6
<211> 1170
<212> DNA
<213> Artificial Sequence

<220>
<223> Description of Artificial Sequence: Synthetic
      construct

<400> 6
atggcacacc atcatcatca tcatcccggg ggatccggtt ctggtaccat ggctattagc 60
gtcactatgg acaatatatt aagcggcttc gaaaacgaat acgacgttat ctacctgaaa 120
ccactggctg gcgtttaccg ttcgctgaaa aaacagattg aaaaaaatat tttcaccttc 180
aacctgaacc tcaatgacat actgaacagc cgactcaaga aacgaaaata ctttctggac 240
gttctggaaa gcgacctgat gcagttcaaa cacatttcga gcaacgaata tattattgaa 300
gacagcttca aactgctcaa cagcgaacag aaaaataccc tcctgaaatc ttataaatac 360
atcaaagaaa gcgttgaaaa cgacattaaa ttcgctcaag aaggcatctc ttactacgaa 420
aaggttctgg ccaaatacaa ggacgacctg gaaagcatta aaaaagttat aaaagaagaa 480
aaggagaagt ttccaagcag cccaccaacc actccaccca gcccagctaa aaccgatgaa 540
cagaagaagg aaagcaagtt tctcccattc ctgaccaata ttgagacgct gtataacaat 600
ctggttaaca aaattgatga ctatctgatt aatctgaagg ctaagattaa tgactgcaac 660
gttgaaaaag acgaagctca cgttaaaatc actaaactct ctgacctgaa agctattgac 720
gataaaatcg acctcttcaa aaatccaacg gattttgaag ctattaaaaa actcatcaac 780
gacgacacga aaaaagacat gctcgggaaa ctgctctcta ccggcctggt tcagaatttc 840
ccaaacacca tcatcagcaa actgattgaa ggcaaatttc aggacatgct gaatattagc 900
cagcatcagt gtgttaaaaa acagtgccca gaaaacagcg gctgctttcg tcacctggac 960
gaacgtgaag aatgcaaatg cctgctgaac tataaacagg aaggcgacaa atgcgttgaa 1020
aacccaaacc caacttgcaa tgaaaacaac ggcggctgcg acgctgacgc caaatgcacg 1080
gaagaagaca gcggctcgaa tggcaagaaa ataacctgcg aatgcactaa accagacagc 1140
tacccactct ttgacggcat tttttgtagc 1170

<210> 7
<211> 371
<212> PRT
<213> Plasmodium falciparum

<400> 7
Met Ala His His His His His His Pro Gly Gly Ser Gly Ser Gly Thr
1               5                   10                  15

Ala Val Thr Pro Ser Val Ile Asp Asn Ile Leu Ser Lys Ile Glu Asn
            20                  25                  30

Glu Tyr Glu Val Leu Tyr Leu Lys Pro Leu Ala Gly Val Tyr Arg Ser
        35                  40                  45

Leu Lys Lys Gln Leu Glu Asn Asn Val Met Thr Phe Asn Val Asn Val
    50                  55                  60

Lys Asp Ile Leu Asn Ser Arg Phe Asn Lys Arg Glu Asn Phe Lys Asn
65                  70                  75                  80

Val Leu Glu Ser Asp Leu Ile Pro Tyr Lys Asp Leu Thr Ser Ser Asn
                85                  90                  95

Tyr Val Val Lys Asp Pro Tyr Lys Phe Leu Asn Lys Glu Lys Arg Asp
            100                 105                 110

Lys Phe Leu Ser Ser Tyr Asn Tyr Ile Lys Asp Ser Ile Asp Thr Asp
        115                 120                 125

Ile Asn Phe Ala Asn Asp Val Leu Gly Tyr Tyr Lys Ile Leu Ser Glu
    130                 135                 140

Lys Tyr Lys Ser Asp Leu Asp Ser Ile Lys Lys Tyr Ile Asn Asp Lys
145                 150                 155                 160

Gln Gly Glu Asn Glu Lys Tyr Leu Pro Phe Leu Asn Asn Ile Glu Thr
                165                 170                 175

Leu Tyr Lys Thr Val Asn Asp Lys Ile Asp Leu Phe Val Ile His Leu
            180                 185                 190

Glu Ala Lys Val Leu Asn Tyr Thr Tyr Glu Lys Ser Asn Val Glu Val
        195                 200                 205

Lys Ile Lys Glu Leu Asn Tyr Leu Lys Thr Ile Gln Asp Lys Leu Ala
    210                 215                 220

Asp Phe Lys Lys Asn Asn Asn Phe Val Gly Ile Ala Asp Leu Ser Thr
225                 230                 235                 240

Asp Tyr Asn His Asn Asn Leu Leu Thr Lys Phe Leu Ser Thr Gly Met
                245                 250                 255

Val Phe Glu Asn Leu Ala Lys Thr Val Leu Ser Asn Leu Leu Asp Gly
            260                 265                 270

Asn Leu Gln Gly Met Leu Asn Ile Ser Gln His Gln Cys Val Lys Lys
        275                 280                 285

Gln Cys Pro Gln Asn Ser Gly Cys Phe Arg His Leu Asp Glu Arg Glu
    290                 295                 300

Glu Cys Lys Cys Leu Leu Asn Tyr Lys Gln Glu Gly Asp Lys Cys Val
305                 310                 315                 320

Glu Asn Pro Asn Pro Thr Cys Asn Glu Asn Asn Gly Gly Cys Asp Ala
                325                 330                 335

Asp Ala Lys Cys Thr Glu Glu Asp Ser Gly Ser Asn Gly Lys Lys Ile
            340                 345                 350

Thr Cys Glu Cys Thr Lys Pro Asp Ser Tyr Pro Leu Phe Asp Gly Ile
        355                 360                 365

Phe Cys Ser
    370


<210> 8
<211> 1113
<212> DNA

<213> Artificial Sequence

<220>
<223> Description of Artificial Sequence: Synthetic
      construct

<400> 8
atggcacacc atcatcatca tcatcccggg ggatccggtt ctggtaccgc tgttacgcca 60
tcggttattg acaatatcct cagcaaaatt gaaaacgaat acgaggttct gtacctgaaa 120
ccactggctg gcgtttaccg ttctctgaaa aaacagctgg aaaacaatgt tatgaccttc 180
aacgttaacg ttaaggacat tctgaacagc cgcttcaaca aacgcgaaaa ctttaaaaac 240
gttctggaaa gcgacctgat tccatacaaa gacctgacca gctctaacta cgttgtcaaa 300
gacccataca aattcctcaa caaagaaaaa cgtgacaaat ttctgtcgtc ttacaactac 360
attaaggaca gcatcgacac ggacatcaac ttcgctaacg acgttctcgg ctactacaaa 420
atcctgtcgg aaaaatacaa aagcgacctg gacagcatta aaaaatacat taatgataaa 480
cagggcgaaa acgagaaata tctccctttc ctgaataaca ttgagacgct gtacaaaacc 540
gttaacgaca aaattgacct gttcgttatt cacctggaag ctaaagttct caactacacc 600
tacgagaaaa gcaatgttga agttaaaatc aaagaactca actatctgaa aaccattcag 660
gataaactcg ctgacttcaa aaaaaacaat aactttgttg gcattgctga cctgagcacc 720
gactacaatc acaacaatct gctcaccaag tttctctcta ccggcatggt tttcgaaaac 780
ctcgctaaaa cggttctgag caacctgctc gacggcaatc tccagggcat gctgaatatt 840
agccagcatc agtgtgttaa aaaacagtgc ccacagaaca gcggctgctt tcgtcacctg 900
gacgaacgtg aagaatgcaa atgcctgctg aactataaac aggaaggcga caaatgcgtt 960
gaaaacccaa acccaacttg caatgaaaac aacggcggct gcgacgctga cgccaaatgc 1020
acggaagaag acagcggctc gaatggcaag aaaattacct gcgaatgcac taaaccagac 1080
agctacccac tctttgacgg cattttctgc agt                              1113

<210> 9
<211> 371
<212> PRT
<213> Plasmodium falciparum

<400> 9
Met Ala His His His His His His Pro Gly Gly Ser Gly Ser Gly Thr
1               5                   10                  15

Ala Val Thr Pro Ser Val Ile Asp Asn Ile Leu Ser Lys Ile Glu Asn
            20                  25                  30

Glu Tyr Glu Val Leu Tyr Leu Lys Pro Leu Ala Gly Val Tyr Arg Ser
        35                  40                  45

Leu Lys Lys Gln Leu Glu Asn Asn Val Met Thr Phe Asn Val Asn Val
    50                  55                  60

Lys Asp Ile Leu Asn Ser Arg Phe Asn Lys Arg Glu Asn Phe Lys Asn
65                  70                  75                  80

Val Leu Glu Ser Asp Leu Ile Pro Tyr Lys Asp Leu Thr Ser Ser Asn
                85                  90                  95

Tyr Val Val Lys Asp Pro Tyr Lys Phe Leu Asn Lys Glu Lys Arg Asp
            100                 105                 110

Lys Phe Leu Ser Ser Tyr Asn Tyr Ile Lys Asp Ser Ile Asp Thr Asp
        115                 120                 125

Ile Asn Phe Ala Asn Asp Val Leu Gly Tyr Tyr Lys Ile Leu Ser Glu
    130                 135                 140

Lys Tyr Lys Ser Asp Leu Asp Ser Ile Lys Lys Tyr Ile Asn Asp Lys
145                 150                 155                 160

Gln Gly Glu Asn Glu Lys Tyr Leu Pro Phe Leu Asn Asn Ile Glu Thr

165                 170                 175

Leu Tyr Lys Thr Val Asn Asp Lys Ile Asp Leu Phe Val Ile His Leu
            180                 185                 190

Glu Ala Lys Val Leu Asn Tyr Thr Tyr Glu Lys Ser Asn Val Glu Val
        195                 200                 205

Lys Ile Lys Glu Leu Asn Tyr Leu Lys Thr Ile Gln Asp Lys Leu Ala
    210                 215                 220

Asp Phe Lys Lys Asn Asn Asn Phe Val Gly Ile Ala Asp Leu Ser Thr
225                 230                 235                 240

Asp Tyr Asn His Asn Asn Leu Leu Thr Lys Phe Leu Ser Thr Gly Met
                245                 250                 255

Val Phe Glu Asn Leu Ala Lys Thr Val Leu Ser Asn Leu Leu Asp Gly
            260                 265                 270

Asn Leu Gln Gly Met Leu Asn Ile Ser Gln His Gln Cys Val Lys Lys
        275                 280                 285

Gln Cys Pro Gln Asn Ser Gly Cys Phe Arg His Leu Asp Glu Arg Glu
    290                 295                 300

Glu Cys Lys Cys Leu Leu Asn Tyr Lys Gln Glu Gly Asp Lys Cys Val
305                 310                 315                 320

Glu Asn Pro Asn Pro Thr Cys Asn Glu Asn Asn Gly Gly Cys Asp Ala
                325                 330                 335

Asp Ala Lys Cys Thr Glu Glu Asp Ser Gly Ser Asn Gly Lys Lys Ile
            340                 345                 350

Thr Cys Glu Cys Thr Lys Pro Asp Ser Tyr Pro Leu Phe Asp Gly Ile
        355                 360                 365

Phe Cys Ser
    370

<210> 10
<211> 6
<212> PRT
<213> Artificial Sequence

<220>
<223> Description of Artificial Sequence: Synthetic
      6xHis tag

<400> 10
His His His His His His
  1               5

<210> 11
<211> 20
<212> PRT
<213> Plasmodium falciparum

<400> 11
Tyr Ile Asn Asp Lys Gln Gly Glu Asn Glu Lys Tyr Leu Pro Tyr Leu
  1               5                  10                  15

Asn Asn Ile Glu
            20

## Description

### STATEMENT AS TO RIGHTS TO INVENTIONS MADE UNDER FEDERALLY SPONSORED RESEARCH AND DEVELOPMENT

This work was supported by the United States Army Medical Research and Materiel Command as well as other Federal Government Agencies. The U.S. government may have certain rights in the invention.

### CROSS-REFERENCE TO RELATED APPLICATIONS

This application claims priority to U.S. provisional application no. 60/823,897, filed August 29, 2006, which is hereby incorporated by reference. The following patent applications are also incorporated herein by reference: U.S. Application Ser. No. 11/428,380 filed June 30, 2006; U.S. application Ser. No. 10/057,531 filed January 25, 2002; U.S. application Ser. No. 10/057,532 filed January 25, 2002; U.S. application Ser. No. 10/404,667 filed April 1, 2003; Ser. No. 10/404,668 filed April 1, 2003, from PCT/US03/10384 filed April 1, 2003 and claiming priority to U.S. Application 60/369,741 filed April 1, 2002, U.S. Application 60/379,688 filed May 9, 2002, and U.S. Application 60/425,719 filed November 12, 2002; and U.S. application Ser. No. 10/677,641 filed October 1, 2003.

### FIELD OF THE INVENTION

The present invention is directed to recombinant DNA technology and immunology, particularly antigens and related vaccines for prevention of malarial infection. The present invention includes novel recombinant malarial proteins, and fragments of the same, that have unique structural and/or chemical properties that allow them to be expressed in greater quantity **in** heterologous systems. These recombinant proteins and fragments have immunogenic properties superior to malarial proteins expressed using different expression systems and coding sequences, but having the same primary amino acid sequence.

### BACKGROUND OF THE INVENTION

Malaria is a devastating disease that causes widespread morbidity and mortality in areas where it is transmitted by anopheline mosquitoes. In areas of high transmission young children and non-immune visitors are most at risk from this disease, which is caused by protozoa of the genus *Plasmodium.* In areas of lower or unstable transmission, epidemics of the disease can result and afflict individuals of all ages. The most dangerous form of malaria, responsible for much of the morbidity and most of the mortality, is caused by the species *Plasmodium falciparum.* It has been estimated that 2 billion people are at risk from malaria, with 200-300 million clinical cases and 1-2 million deaths each year.

The parasite has a complex life cycle in its human and mosquito hosts. In humans the stage of the life cycle which is responsible for the clinical symptoms of the disease occurs in the bloodstream. During this phase the parasite is largely hidden within host red blood cells. Here the parasite grows and multiplies. For example, within a red blood cell each *P. falciparum* parasite divides several times to produce approximately 20 new ones during a 48 hour cycle. At this point the red blood cell is burst open and the parasites (called merozoites at this stage) are released into the bloodstream. The merozoites must enter new red blood cells in order to survive and for the cycle of replication in the blood to continue. If the parasites do not manage to enter red blood cells they cannot survive for very long and are rapidly destroyed. Symptoms of malaria such as fever are associated with this cyclic merozoite release and re-invasion of red blood cells.

There is an urgent need for a vaccine against malaria. There is no effective vaccine currently available. In addition, mosquito control by the spraying of residual insecticides is either becoming ineffective or considered to be unacceptable, and there is a very worrying spread of drug resistance within parasites. The rapid spread of drug resistance is worrying because compounds such as the cheap and once-effective chloroquine are no longer useful in many parts of the world, and there are few if any new drugs available that are both cheap and effective. Vaccines against microorganisms can be very cost effective and efficient ways to protect populations against infectious diseases.

Because of the complexity of the parasite's life cycle there are a number of points in its development within humans that could be the target of a protective immune response. It is known that with increasing age and exposure individuals do become immune to malaria, suggesting that protective responses do develop with time. Broadly speaking there are three types of vaccine strategy: to target the pre-erythrocytic stages, the asexual blood stage and the sexual stage. The pre-erythrocytic stages are the spocozoites that are injected by an infected mosquito when it takes a blood meal and the initial development of the parasite in the liver. The asexual blood stage is the infection and release of merozoites from red blood cells that occurs in a cyclic manner, and the stage responsible for the manifestation of the clinical symptoms. The sexual stage takes place in the mosquito's gut after it has ingested gametocytes in a blood meal and this initiates the infection of the insect to complete the cycle; a vaccine against the sexual stages would not protect the individual but could reduce transmission and therefore the incidence of malaria in a given human population.

During the asexual cycle in the blood the parasite is directly exposed to the host's immune system, and in particular to antibodies circulating within the bloodstream, only transiently: when merozoites are released by rupture of one cell and before they penetrate another. If there are specific antibodies that can bind to the surface of the parasite then it is possible that these antibodies will interfere with the ability of the parasite to invade a new red blood cell. In fact it has been shown that several monoclonal antibodies that recognize single epitopes on parasite surface proteins, are capable of neutralizing the parasite and preventing the cycle of reproduction within red blood cells.

One of the best characterised proteins on the surface of the merozoite is called merozoite surface protein 1 (MSP-1). MSP-1 is a large protein that varies in size and amino acid sequence in different parasite lines. It is synthesized as a precursor molecule of 200 kDa by the intracellular parasite and located on the parasite's surface. During release of merozoites from red blood cells and the re-invasion of new erythrocytes the protein undergoes at least two proteolytic modifications. In the first modification as a result of a process called primary processing, the precursor is cleaved to four fragments of 83, 30, 38 and 42 kDa that remain together as a complex on the merozoite surface. This complex also contains two other proteins of 22 kDa and 36 kDa derived from different genes. The complex is maintained by non-covalent interactions between the different subunits and is held on the merozoite surface by a glycosyl phosphatidyl inositol anchor, attached to the C-terminus of the 42 kDa fragment and inserted into the plasma membrane of the merozoite. At the time of merozoite invasion of an erythrocyte the C-terminal 42 kDa fragment is cleaved by a second proteolytic cleavage in a process called secondary processing. The result of secondary processing is that the entire complex is shed from the surface of the merozoite except for a C-terminal sub-fragment that consists of just under one hundred amino acids and which is carried into the newly invaded erythrocyte on the surface of the merozoite.

### SUMMARY OF THE INVENTION

Difficulties with recombinant protein expression in *Escherichia coli,* such as low expression or formation of insoluble aggregates, are often attributed to a discordant pattern of synonymous codon usage between *E. coli* and the heterologous gene's natural host. Such patterns of codon usage may be particularly important during translation of relatively unstructured polypeptide "link/end" segments, which separate structural elements, and where ribosomal progression may slow as the ribosome encounters codons that are infrequently used in the natural host. To approximate native patterns of codon usages, we developed an algorithm that identifies putative link/end segments in proteins, and adjusts the use of *E*. *coli* codons in such regions to reflect the frequency of codon usage seen in nature; i.e., if a domain contains an infrequently-used codon for threonine in the natural host, a synonymous codon that is infrequently used in *E*. *coli* is substituted.

This codon harmonization algorithm was applied to the heterologous expression of several *Plasmodium falciparum* proteins including the FVO, 3D7 and Camp alleles of MSP1₄₂, which are the C-terminal 42 kDa fragments from a family of major merozoite surface proteins (MSP1), and the 3D7 allele of the LSA-NRC fragment from the hepatic stage antigen LSA1. In each case, expression of the "recoded" gene exceeded that of the native gene by 4- to 1,000-fold, producing soluble protein in quantities that are suitable for vaccine manufacture. The recombinant MSP1₄₂ proteins reacted well with a variety of parasite-induced monoclonal antibodies (mAb) that are specific for structural epitopes and induced antibodies that were functionally active against parasites *in vitro.* These results suggest that the recombinant proteins expressed from codon harmonized genes fold properly, assuming native conformations. Codon harmonization may further provide a general strategy for improving the expression of soluble functional proteins during heterologous expression in hosts other than *E. coli.*

### BRIEF DESCRIPTION OF THE DRAWINGS

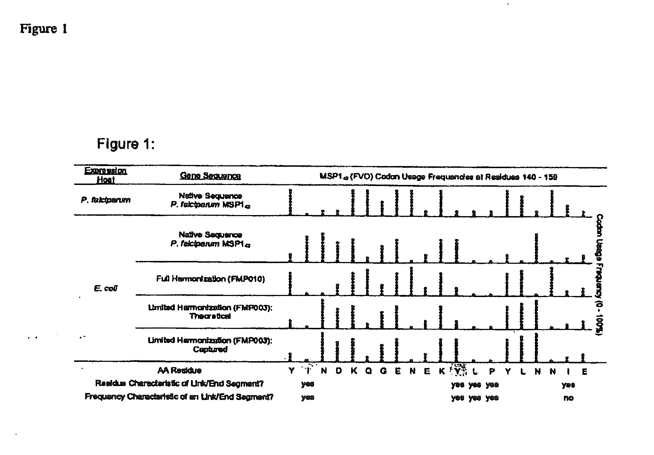

Fig. 1: Comparison of Codon Usage Frequencies in the Native MSP1₄₂ (FVO) and Recoded Sequences as a Function of Expression Host, for either native *P. falciparum* or *E. coli* expression. Amino acid segment of MSP1₄₂ (FVO) sequence is shown (residue # 140-159) (SEQ ID NO: 11) with the codon usage frequencies rounded to the nearest 10%. The shaded residue's shown, I(141) and Y(151), are predicted to be highly discordant for expression in *E. coli.*

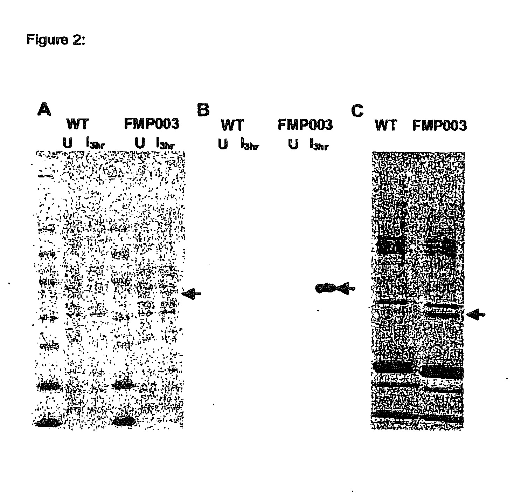

Fig. 2: Comparison of MSP1₄₂ (FVO) expression from plasmids encoding either wild type or FMP003 sequence by SDS-PAGE and Coomassie Blue staining or Western Blotting. Panel A, Coomassie Blue stained gels at the time of induction (U) or 3 hours post induction (I₃ₕᵣ); Panel B, Western blots at the time of induction (U) or 3 hours post induction (I₃ₕᵣ); Panel C, Coomassie Blue stained gels of protein following affinity purification on Ni⁺²-NTA chromatography. Arrows indicate migration of MSP1₄₂ (FVO).

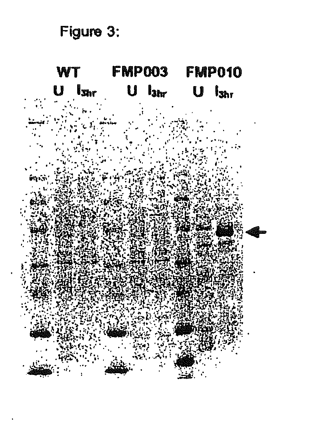

Fig. 3: Comparison of expression levels from wild type (WT) and full gene codon harmonized MSP1₄₂ (FVO), (FMP010). Total *E. coli* cell lysates were separated by SDS-PAGE followed by staining with Coomassie Blue at either the time of induction (U) or 3 hours post induction (I₃ₕᵣ) with 0.1 mM IPTG. The arrow indicates the migration of MSP1₄₂ (FVO).

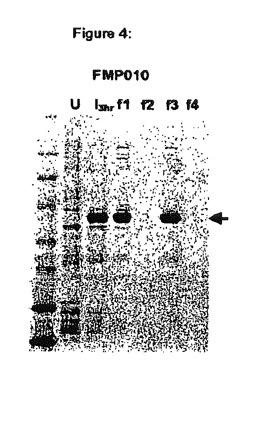

Fig. 4: Sarkosyl treatment and solubility; U, Un-induced FMP010 cell lysate; I₃ₕᵣ, FMP010 cell lysate following 3 hours of induction with 0.1 mM IPTG; f1, 30,000 x g supemate; f2, 30,000 x g pellet, f3; 100,000 x g supemate, f4; 100,000 x g pellet,. The arrow indicates the migration of MSP1₄₂ (FVO).

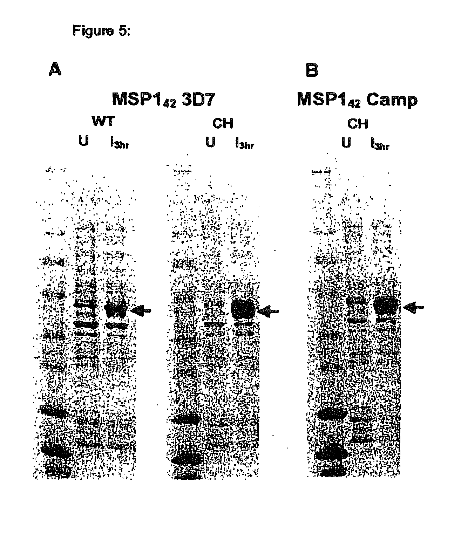

Fig. 5: Comparison of expression levels of wild type (WT) and full gene codon harmonized (CH) MSP1₄₂ gene fragments from 3D7 and Camp strain *P. falciparum.* Total E. *coli* cell lysates collected at time zero (uninduced =U) or 1, 2, or 3, hours (I ₁ₕᵣ, I ₂ₕᵣ I ₃ₕᵣ, respectively), after induction with 0.1 mM IPTG. Samples were separated by SDS-PAGE and stained with Coomassie Blue. Panel A; MSP1₄₂ (3D7) from 3D7 strain *P. falciparum:* expression of wild type (WT) and codon harmonized (CH) genes respectively. Panel B; MSP1₄₂ (Camp) from Camp strain *P. falciparum:* expression of codon harmonized (CH) gene. The arrows indicate the migration of MSP1₄₂.

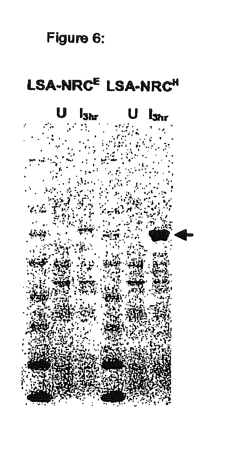

Fig. 6: Evaluation of expression levels for the *E. coli* codon optimized LSA-NRC^{E} (3D7) and full gene codon harmonized LSA-NRCH (3D7). Total *E. coli* cell lysates were separated by SDS-PAGE followed by staining with Coomassie Blue at the time of induction (U) and 3 hours post induction (I ₃ₕᵣ) with 0.1 mM IPTG. The arrow indicates the migration of LSA-NRC.

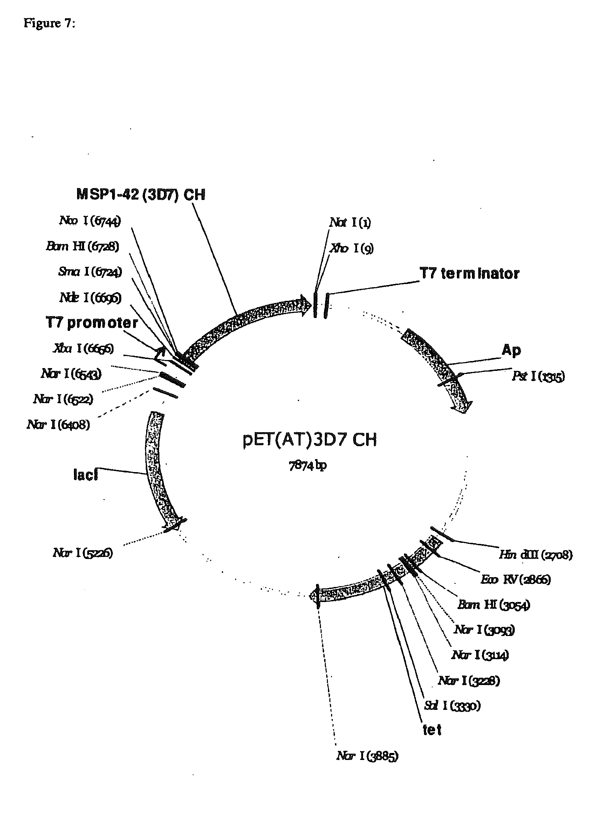

Fig. 7: Plasmid Map: *P. falciparum* MSP1-42 3D7 CH.

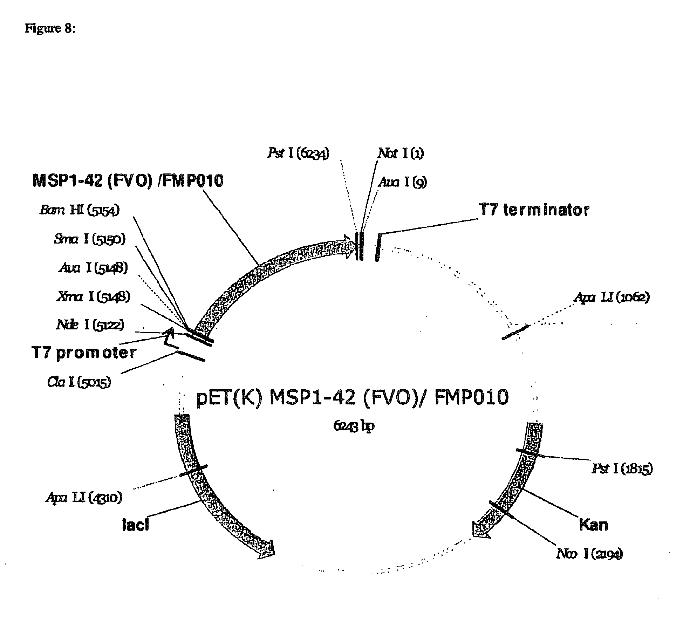

Fig. 8: Plasmid Map: *P. falciparum* MSP1-42 FVO CH/FMP010.

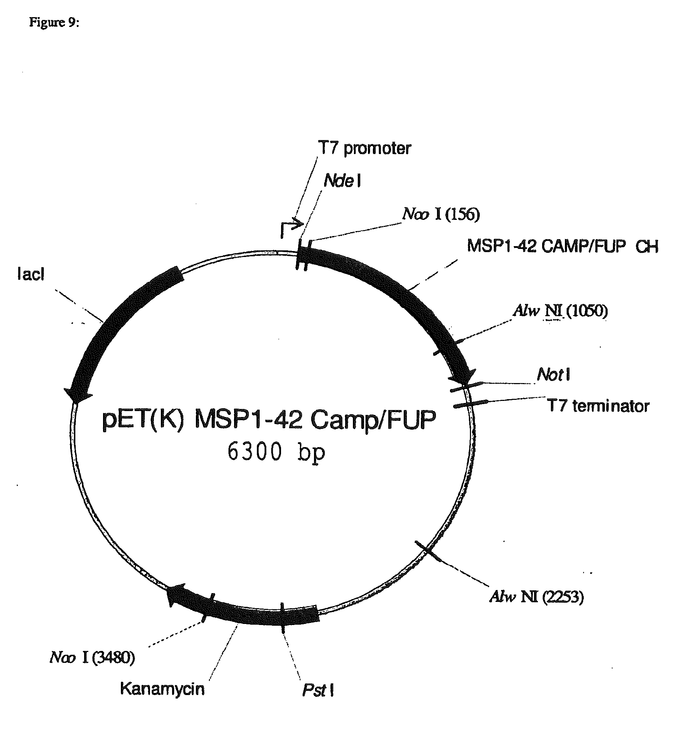

Fig. 9: Plasmid Map: *P. falciparum* MSP1-42 Camp/FUP CH.

### DEFINITIONS

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them unless specified otherwise.

"Natural host" refers to a cell that is expressing a protein from a nucleic acid transcript that is naturally present in the host.

A molecule, more particularly for the present invention a protein or nucleic acid is "native to a natural host" when the molecule is normally part of the natural host in the wild, and is not introduced to the host through molecular biological techniques.

"Non-natural host" or "heterologous host" refers to a cell that is expressing a protein from a nucleic acid transcript that is not naturally found in the natural host, as occurs in recombinant cells formed through molecular biological techniques.

"Coding region" refers to the portion of a nucleic acid transcript or gene that can be translated into polypeptide or protein when operably linked to expression control sequences and introduced to a suitable translation system.

"Translation system" refers to an enzyme-based system capable of producing a protein de novo when a nucleic acid transcript is introduced into the system.

"Contiguous codons" refers to two or more codons linked in frame with each other without any intervening nucleotides.

"Synonymous codons" are sets of codons whose corresponding tRNAs are charged with a common amino acid such that, when appearing in a transcript, the amino acid positions corresponding to synonymous codons are occupied by the same amino acid.

"Synonymous coding sequences" refers to coding regions that differ in nucleic acid base sequence, but encode the same protein or peptide: i.e., peptides produced from the synonymous coding sequences have identical amino acid sequences, but may differ in higher order structure (secondary, tertiary, etc ... ).

"Selective translational attenuation" refers to a process by which certain codons of a nucleic acid transcript are substituted with synonymous codons in order to affect the rate of protein translation.

The "codon usage frequency" for a cell or organism is calculated by determining the frequency at which a given synonymous codon is used in cellular transcripts relative to other synonymous codons corresponding to the same amino acid. Codon usage frequency is defined as the "percentage usage of a given synonymous codon within that set of synonymous codons". For instance, the global usage frequencies in Homo sapiens for the four codons encoding the amino acid valine (GTT, GTC, GTA and GTG), are 11, 14.6, 7.2 and 28.4 codons, respectively, per 1000 codons in all human transcripts. By summing the global usage frequencies for these four codons (a total of 48.2 per 1000) the individual codon usage frequencies can be obtained simply by divided this value by that seen for a specific codon. Therefore, within this set of synonymous codons, the usage frequencies calculated for each codon are 18%, 23.9%, 11.8% and 46.4%, respectively.

To more easily compare usage frequencies between different sets of synonymous codons, the term "relative codon usage" can be applied, which takes into account the differing number of members in sets of synonymous codons (e.g., 2, 4 or 6 codons); this comparative value has also been called "relative synonymous codon usage" or RSCU (Sharp, P. M., and W. H. Li, (1987). Nucleic Acids Research 15: 1281-1295). To calculate this value, one divides the codon usage frequency for an individual codon (converted to a percentage--0.18=18%) by the arithmetic mean value (as a simple fraction) for that set of codons and then subtracts the value of 100; for the valine codon GTT in the example above, this would be (18/0.25)-100 or -28%. Relative codon usage, then, is the "percentage deviation from the mean" for a given codon group, i.e., if the arithmetic mean usage for valine (4 codons) is 25% (zero deviation), then a codon usage frequency of 18% for a valine codon (GTT, in the example above) would represent a negative deviation from the mean of 28% or a relative codon usage of -28%; likewise, a codon usage frequency of 46% for a valine codon (GTG, in the example above) would represent a positive deviation from the mean of 86% or a relative codon usage of +86%.

A "translationally-harmonized nucleic acid" or "harmonized nucleic acid" is any nucleic acid that encodes a protein and has been modified to improve soluble levels of the protein when the nucleic acid is expressed in a non-natural host by at least 10%, more preferably 20%, advantageously more than 30%, ideally more than 40% over the levels achieved when expressing the unmodified nucleic acid in the non-natural host under the same conditions, where the modifications are substitutions of selected codons with synonymous codons to achieve selective translational attenuation.

A "non-harmonized nucleic acid" is any nucleic acid that encodes a protein and has not been modified to improve soluble levels of the protein when the nucleic acid is expressed in a non-natural host, where the modifications are substitutions of selected codons with synonymous codons to achieve selective translational attenuation.

"Expression control sequences" are those nucleotide sequences, both 5' and 3' to a coding region, that are required for the transcription and translation of the coding region in a host organism. Regulatory sequences include a promoter, ribosome binding site, optional inducible elements and sequence elements required for efficient 3' processing, including polyadenylation. When the structural gene has been isolated from genomic DNA, the regulatory sequences also include those intronic sequences required for splicing of the introns as part of mRNA formation in the target host.

"Nucleic acid" refers to deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2'-0-methyl ribonucleotides, peptide-nucleic acids (PNAs).

Unless otherwise indicated, a particular nucleic acid sequence also implicitly encompasses conservatively modified variants thereof (e.g., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); Rossolini et al., Mol. Cell. Probes 8:91-98 (1994)). The term nucleic acid is used interchangeably with gene, cDNA, mRNA, oligonucleotide, and polynucleotide.

The term "amino acid" refers to naturally occurring and synthetic amino acids, as well as amino acid analogs and amino acid mimetics that function in a manrier similar to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, i.e., a carbon that is bound to a hydrogen, a carboxyl group, an amino group, and an R group, e.g., homoserine, norleucine, methionine sulfoxide, methionine methyl sulfonium. Such analogs have modified R groups (e.g., norleucine) or modified peptide backbones, but retain the same basic chemical structure as a naturally occurring amino acid. Amino acid mimetics refers to chemical compounds that have a structure that is different from the general chemical structure of an amino acid, but that function in a manner similar to a naturally occurring amino acid.

Amino acids may be referred to herein by either commonly known three letter symbols or by the one-letter symbols recommended by the IUPAC-IUB Biochemical Nomenclature Commission. Nucleotides, likewise, may be referred to by their commonly accepted single-letter codes.

The term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all.

The terms "identical" or percent "identity," in the context of two or more nucleic acids or polypeptide sequences, refer to two or more sequences or subsequences that are the same or have a specified percentage of amino acid residues or nucleotides that are the same (i.e., 60% identity, 65%, 70%, 75%, 80%, preferably 85%, 90%, 91%, 92%, 93%, 94%, 95%, 96%, 97%, 98%, 99% or higher identity to a reference sequence), when compared and aligned for maximum correspondence over a comparison window, or designated region as measured using one of the following sequence comparison algorithms or by manual alignment and visual inspection.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. For sequence comparison of translationally-harmonized nucleic acid sequences and the polypeptides they encode, the BLAST and BLAST 2.0 algorithms and the default parameters discussed below are used.

A "comparison window", as used herein, includes reference to a segment of any one of the number of contiguous positions in which a sequence may be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by manual alignment and visual inspection (see, e.g., Current Protocols in Molecular Biology (Ausubel et al., eds. 1995 supplement)).

A preferred example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990), respectively. BLAST and BLAST 2.0 are used, with the parameters described herein, to determine percent sequence identity for the nucleic acids and proteins of the invention. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., supra). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (see Henikoff & Henikoff, Proc. Natl. Acad. Sci. USA 89:1091 (1989)) alignments (B) of 50, expectation (E) of 10, M=5, N=-4, and a comparison of both strands.

The BLAST algorithm also performs a statistical analysis of the similarity between two sequences (see, e.g., Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The term "operably linked" refers to a linkage of polynucleotide elements in a functional relationship. With regard to the present invention, the term "operably linked" refers to a functional linkage between a nucleic acid expression control sequence (such as a promoter, or a ribosome binding site) and a second nucleic acid sequence, e.g., wherein the expression control sequence directs or initiates translation of the nucleic acid corresponding to the second sequence and, in some instances, itself. Thus, a nucleic acid is "operably linked" when it is placed into a functional relationship with another nucleic acid sequence.

### DETAILED DESCRIPTION

The current invention provides novel nucleic acid coding sequences that express recombinant proteins and peptides with superior properties to the same proteins and peptides expressed from alternative nucleic acid sequences, including native coding sequences, in a heterologous host. Using the techniques and sequences described herein, one of ordinary skill in the art may produce recombinant protein in more abundance, that is folded in higher order structure more analogous to the native protein than other recombinant proteins, and/or is more immunogenic than recombinant proteins produced using alternative methods and coding sequences.

It will be appreciated that the coding sequences described herein need not be used in their entirety to produce a protein with the desired secondary structure suitable for immunogenic, structural or other utility. Indeed any contiguous stretch of nucleotides sufficient to produce the desired protein or peptide fragment may be taken from the sequences described herein, provided that the selected coding region for the protein or peptide fragment contains at least one, preferably 2, 3, 4, 5, 6, 7, 8, 9 or more nucleotide base changes made in the native coding region to produce the coding region for the desired protein or peptide fragment of the invention. Preferably such coding regions encode peptides or proteins at least 8 more preferably 10, 12, 15, 20, 50 100, 1000 or more amino acyl residues in length.

It will also be appreciated by one of ordinary skill in the art that the present invention may be practiced using any non-native host including any prokaryotic or eukaryotic cell. Indeed, the present invention may also be used with any viral expression system, regardless of cell type, provided that viral protein expression is at least partially controlled through translational and/or transcriptional modulation mediated through the nucleic acid sequence of the coding region being expressed. Of course in such viral expression systems the coding region of the invention will be operably linked to suitable viral control sequences, as will be appreciated by those of skill in the art. The preferred expression system of the present invention are strains of *E. coli* as described below.

Recoding gene sequences using the present invention provides optimal expression in a heterologous host leading to a highly expressed, soluble and more correctly folded protein.

This invention can be used to optimize translation kinetics and protein folding within heterologous expression systems, i.e. prokaryotic and eukaryotic. The application of this invention can lead to modulation of translation kinetics (rates) and proper protein folding during heterologous protein synthesis, thus yielding high levels of soluble protein product.

Species-specific disparities in codon usage are frequently cited as the cause for failures in recombinant gene expression by heterologous expression hosts. Such failures include lack of expression, or the expression of protein that is non-functional or insoluble, or protein that is fragmented owing to proteolysis or premature termination of translation^{1;2}. All but the last of these failures are attributable to misfolded protein. In *Escherichia coli* as well as eukaryotic species, nascent proteins fold co-translationally within the ribosomal tunnel, which is both a protective environment within which secondary structure begins to form^{3; 4; 5}, and a dynamic environment that influences nascent protein structure^{6; 7; 8; 9}. In addition to the influence of the ribosomal tunnel, variations in the rate of mRNA translation may also play a role in developing secondary structure in the nascent protein. Translation is not a steady state process, rather it occurs in pulses, as can be observed from ribosomal pausing and even ribosome stacking, on specific stretches of mRNA¹⁰; these temporal changes in translational rate depend on relative tRNA levels¹¹.

Based on the concepts that protein synthesis and folding in *E. coli* is co-translational and that nucleotide sequence-dependent modulation of translation kinetics might influence nascent polypeptide folding - we developed a strategy to recode a target gene sequence for heterologous expression in *E. coli* by substituting native codons with synonymous ones having the same or similar usage frequencies in the expression host. In this approach, termed "codon harmonization," synonymous codons from *E. coli* were selected that match as closely as possible the codon usage frequency used in the native gene (either equal to, higher or lower usage frequency), unless empirical structure calculations showed the codons to be associated with a putative link/end segment. Link/end segments were recoded by selecting the closest matching synonymous *E. coli* codon having a usage frequency equal to or less than that of its respective isoacceptor codon's usage frequency in the native gene host.

Disharmony between codon usage patterns for *Plasmodium falciparum* malaria parasite target genes and *E. coli* has proven particularly challenging for heterologous expression in *E. coli* owing to the 80% AT bias in the structural genes from *P. falciparum*²⁰*.* We applied this algorithm initially, in a limited way (one codon change in a predicted link/end segment) and later to the full gene sequence of the *P. falciparum* MSP1₄₂ (FVO) antigen, and, then to three additional malaria vaccine candidates MSP1₄₂ (3D7), MSP1₄₂ (Camp), and LSA-NRC (3D7). In every case, both protein yield and protein solubility improved significantly. For the four fully harmonized gene sequences, yields increased to from 3-12 mg purified protein/g wet cell paste, enabling them to be developed for clinical testing as malaria vaccines.

### RESULTS

### Codon Harmonization Algorithm

The identification of transcript regions that are likely to promote ribosomal pausing requires a means to predict such "link/end" segments. We decided to evaluate coding sequence for the presence of those amino acids most often associated with such regions, and to apply a standardized computation to determine operationally if such a region contains infrequently used codons. Table 1 shows the calculated threshold value for infrequent codon usage for these residues.

**Table 1:**

| **Native Host Expression (*P. falciparum)* Residues Typically Found in Link/End Segments** | | | | | | | | |
|---|---|---|---|---|---|---|---|---|
| Residue | Codon A | Codon B | Codon C | Codon D | Codon E | Codon F | | Median Usage Rate |
| Cys | 49.0% | 51.0% | | | | | | 50.0% |
| His | 17.0% | 83.0% | | | | | | 50.0% |
| Ile | 0.1% | 17.0% | 83.0% | | | | | 17.0% |
| Leu | 0.1% | 2.0% | 3.0% | 4.0% | 7.0% | 83.0% | | 3.5% |
| Pro | 0.1% | 8.0% | 15.0% | 77.0% | | | | 11.5% |
| Ser | 2.0% | 3.0% | 4.0% | 20.0% | 34.0% | 37.0% | | 12.0% |
| Thr | 4.0% | 7.0% | 35.0% | 55.0% | | | | 21.0% |
| Trp | 100% | | | | | | | 100% |
| Tyr | 25.0% | 75.0% | | | | | | 50.0% |
| Val | 7 0% | 16.0% | 26.0% | 51.0% | | | | 21.0% |
| | | | | | | | | 33.6% |

The threshold calculation (average of the median usage value for the ten relevant amino acids) was biased toward lower usage frequency by calculating the median usage value for each relevant amino acid rather than its mean usage value. For *P. falciparum* the threshold value was calculated to be 33.6%. A potential link/end segment was calculated to be present if within a 15-residue range two or more codons existed that encoded at least one of the ten relevant amino acids, i.e. those that appear most often in link/end segments, and their usage frequencies were less than 33.6% (averaged from three contiguous codons). Fig. 1 shows an example of a region of the FVO allele of the MSP1₄₂ gene fragment that was calculated to be a link/end segment (top line). Nine of the 20 amino acids shown in this diagram are encoded by isoacceptor codons that are used 20% of the time or less in the native expression host, and among these, four (I141, Y151, L152, P153) belong to the group of ten residues that appear most frequently in the link/end segments of *E*. *coli* proteins. Although I158 satisfies the criterion of being among these ten residues, its isoacceptor usage frequency in the native expression host is greater than 33.6%; therefore this residue is not calculated to be part of the link/end segment. Introduction of the native *P. falciparum* gene sequence into *E. coli* results in a dramatic shift in usage frequencies distributed over the length of the sequence (compare first and second lines). Of the nine low frequency codons identified in the first line, five are shifted to higher usage frequencies that exceed the threshold; I141 and Y151 are among these. In addition several codons that are used at high frequency in the native host are used at low frequency in *E. coli,* namely Q145, N148, Y154, L155, and N157. The usage frequency for the link/end segment residue P153 (CCC) drops from 11% in *P. falciparum* to 0.1% in *E. coli.* Following full codon harmonization of this segment for expression in *E. coli* (i,e., matching the frequency of codon usage at each residue), codon usage patterns for heterologous expression are in good agreement with patterns for expression of the native gene in the native host (compare first and third line), with the usage rate for P153 increasing to 8% as compared to 11% in *P. falciparum.* Our initial studies evaluated the utility of this codon harmonization algorithm by focusing on this region of the gene because, of all of the putative link/end segments that were identified, this was the most discordant when its codon usage patterns were compared for native and heterologous expression.

### Single Codon Application of Codon Harmonization

We wanted to test the value of the algorithm by focusing only on relevant residues in the native sequence that should comprise a link/end segment and whose usage frequency during heterologous expression would exceed the threshold value of 33.6%. Thus we determined that mutating codons for 1141 (ATC→ATA) and Y151 (TAC→TAT) would be sufficient to meet this objective (Fig. 1, compare first and fourth line, also shaded boxes on AA residue line). Although we isolated a mutant that contained the 1141 (ATC→ATA) conversion, we did not capture any mutants containing an Y151 (TAC→TAT) conversion (Fig. 1, fifth line). *E. coli* transformed with the gene, containing the single I141 (ATC→ATA) conversion (designated FMP003) expressed significantly more protein than was obtained from the wild type gene. Although, we were not able to detect expression of the protein from the codon harmonized directly in cell lysates on gels stained with Coomassie Blue (Fig 2A), however it was detected by western blotting while expression of protein from the wild type gene was not (Fig 2B). Expression of FMP003 allowed for isolation of at least 10-fold more protein per gram of wet cells than did expression of the wild type gene sequence (Fig. 2C). The purified FMP003 protein was determined to be soluble based on its presence in the supernatant fraction after centrifugation at 100,000 x g for 1 h (data not shown).

### Full Gene Codon Harmonization

The algorithm was further tested by preparing a "fully harmonized" MSP1₄₂ (FVO) gene fragment (designated FMP010) that was recoded to enable efficient translational pausing during expression in *E. coli.* Densitometry of the gel shown in Fig. 3 shows that after induction with IPTG, expression levels obtained from the harmonized gene sequence, FMP010, exceeded the wild type sequence by at least 100-fold. Protein in the cell lysate after microfluidization was fully soluble in the presence of 0.3% sarkosyl, remaining in the supernatant fraction after sequentially centrifuging at 30,000 and 100,000 x g for 1 h (Fig. 4, f1 and f3, respectively), and was absent from the corresponding pellets (Fig 4, f2 and f4, respectively.).

Since we observed a significant increase in total expression for the FMP010 protein following application of this algorithm to the full gene, we decided to apply the same method to the expression of MSP1₄₂ (3137) and MSP1₄₂ (Camp), which are alternative alleles to FVO. Three hours after IPTG induction, expression levels of MSP1₄₂ (3D7) from the codon harmonized gene were significantly higher than those produced from the native sequence (Fig. 5, CH and WT, respectively, lanes 3), giving approximately a four-fold increase in product yield from 0.8 mg/g wet cells for the native sequence to 3.0 mg/g wet cells from the codon harmonized sequence. Basal levels of expression prior to induction are shown in lanes marked U (un-induced cells). The Camp allele of MSP1₄₂ is a chimera of the FVO and 3D7 alleles. The FVO and 3D7 alleles have different protein sequences (58% identity)²¹ within the N-terminal 300 residues of the protein, known as MSP1₃₃, but differ only by four single amino acid changes within the mature C-terminal 96 amino acid fragment known as MSP1₁₉ (Q14/ E14, K61/T61, N70/S70, and G71/R71, respectively; numbering relative to the MSP1₁₉ fragment). The Camp allele is identical to the 3D7 allele within the MSP1₃₃ domain and the N-terminal 80% of MSP1₁₉, including E14, but changes to FVO (K61, N70, and G71) for the C-terminal 20% of MSP1₁₉. Expression results for codon harmonized MSP1₄₂ (Camp), at 12.0 mg/g wet cells (Fig 5, Panel B) were dramatic compared with expression of wild type MSP142 (Camp), where no expression was detected (not shown).

Expression of the codon harmonized LSA-NRC^{H} malaria vaccine candidate, which has been described elsewhere²², has proven to be the best approach, to date, for the production of this protein in *E. coli.* Expression of native *P. falciparum* sequence encoding the individual N-terminal or C-terminal domains of LSA-1 alone, gives poor yields and unstable plasmids. The same is true for the LSA-NRC^{E} gene, a synthetic gene that was "codon optimized" by substituting with codons that are used frequently in *E. coli* proteins (Hillier, Ware, et al 2005). For this later construct, plasmid loss was especially problematic during exponential growth phase, suggesting that the expression of the product induced a host cell stress response. On the other hand, the synthetic gene designed by using codon harmonization (LSA-NRC^{H}) yielded a dramatic increase in expression levels when compared with LSA-NRC^{E} (Fig. 6, Lane I). Furthermore, the protein was soluble, even in the absence of sarkosyl, and the problem of plasmid retention during exponential growth was corrected. These results further support the hypothesis that the LSA-NRC^{E} and LSA-NRC^{H} protein products folded differently when expressed in *E. coli* and that the expression of LSA-NRC^{H} protein did not induce a deleterious host cell stress response.

### DISCUSSION

Many factors impact translation kinetics including mRNA stability, mRNA secondary structure, tRNA availability, and codon usage^{23; 24}. A role for selective codon usage affecting translational pausing in *E. coli* is supported by comparing proteins of known tertiary structure with their corresponding genes. Such global comparisons across many genes showed that *E. coli* protein domains are as large as 130 residues long¹⁶ and are composed of smaller structural elements that are thirty to sixty residues long¹⁸. These structural elements are separated from one another by unstructured protein link/end segments approximately fifteen residues long and are encoded by clusters of infrequently-used codons^{18; 25} that are separated by 1-10 codons¹⁹. Link/end segments, are thought to be regions for translational pausing, and separate the 30-60 residue structural elements that comprise protein domains as large as 130 residues in *E. coli* ¹⁶. One ribosome translation model predicts that as few as two consecutive infrequently used codons can change the steady-state density of ribosomes on mRNA, causing them to accumulate behind predicted translational pause sites²⁶. Thus reduced translation transit-time through link/end segments may be affected through even minor changes in codon usage and these might facilitate separate folding events for the preceding regions, which are comprised of ordered structure.

High-resolution electron micrographs of the 70S ribosome from *E. coli* show that the 50S subunit contains a bifurcating tunnel that is 85Å to 110Å in length as measured from the amino peptidyl transferase center to the exit site on the distal surface^{27; 28}, which can accommodate nascent peptides of 30 to 72 amino acids, depending on the secondary protein structure ⁴. The opening of the tunnel at the ribosomal surface, which is 25-30Å in diameter, appears to accommodate chaperones, such as Trigger Factor^{29; 30}, which, as necessary³¹, can interact with partially folded nascent polypeptides to promote their complete folding as they are extruded from the tunnel.

A domain that is 130 residues long is too long to remain fully sequestered within the ribosomal tunnel even if it has acquired its secondary structure. However the structural elements, which are 30-60 residues long¹⁶, can be accommodated by the tunnel. As such, translational pausing may be an important co-translational event, which allows time for each structural element within a domain to develop its appropriate secondary structure prior to exiting the ribosomal tunnel. Such a kinetic maturation process can be envisioned to have two effects: a) to allow the C-terminal half of the nascent domain to complete development of its secondary structure while still within the protective environment of the tunnel, prior to the beginning of synthesis of the next structural element and b) to place the N-terminal half of the folded nascent domain at the opening of the ribosomal tunnel, where it might begin to develop tertiary structure, with the help of chaperones, if necessary³¹.

The benefits derived by this rational process of codon substitution are most dramatically shown by our limited mutagenesis to create a single targeted synonymous mutation for I141 within a putative link/end segment in MSP1₄₂ (FVO). Making this single base change, (i.e., to produce FMP003), increased yields of soluble product to approximately 50 pg protein/g of wet cell paste, this being at least ten-fold over what was achieved with the native sequence. The FMP003 antigen was subsequently produced under GMP conditions and shown to be highly immunogenic and efficacious against malaria challenge in an *Aotus* monkey study³². Consistent with this finding, a prior study showed that single synonymous codon substitutions that change codon usage frequencies in link/end segments from infrequent to frequent usage can deleteriously affect enzyme activity³³.

The yield of 50 *µ*g of producng wet cell paste, with FMP003 was too low to be of practical use for vaccine development; therefore we decided to "harmonize" codons throughout the entire gene sequence for MSP1₄₂ (FVO), producing FMP010. Full codon harmonization increased product yield by sixty-fold over that obtained for FMP003. Comparison of the antigenic properties of FMP003 and FMP010 shows that FMP010 was superior for inducing antibodies that inhibit malaria parasite growth *in vitro* (data not shown; the expression, purification and pre-clinical evaluation for this vaccine candidate will be described elsewhere) suggesting that FMP010 is better than FMP003 in terms of replicating the protein structure as it is produced by the malaria parasite.

Our efforts to produce MSP1₄₂ proteins from fully harmonized genes of 3D7 and Camp alleles were as successful in producing large amounts of soluble protein as our efforts with the FVO allele. The success with the Camp allele is notable, as no recombinant protein was detected when the native gene sequence was used for heterologous expression. Recombinant proteins for these two alleles are currently being evaluated in pre-clinical studies to determine their vaccine potential.

Yields of soluble protein from the "codon harmonized" LSA-NRC^{H} gene were significantly greater than from the "codon optimized" LSA-NRC^{E} gene, which was synthesized according to the strategy of replacing every codon synonymously with the highest usage ("optimal") *E. coli* codon. The primary problem that is corrected by codon harmonization is plasmid loss during exponential growth, which suggests that the LSA-NRC^{E} expression product induced a deleterious host cell response; therefore, the proteins expressed from the two synthetic genes must possess significantly different structures. The final protein product from the codon harmonized LSA-NRC^{H} gene has been manufactured under GMP and is currently being tested clinically in malaria naive adult volunteers.

The improved expression of soluble protein was not a consequence of simply changing the G/C ratio in the *P. falciparum* target genes. As shown here, codon optimization of the LSA-NRC^{E} gene fragment for expression in *E. coli* allowed for the production of little if any protein. Others have successfully used *E. coli* to express high levels *of P. falciparum* MSP1₄₂ protein from codon optimized genes, but these proteins were insoluble and required refolding in *vatro*^{34; 35}.

Codon harmonization appears to offer excellent prospects for design and expression of heterologous proteins, at least in *E. coli;* whether or not it will be useful for other expression hosts remains to be determined. If adjustments for relative codon usage can be used to improve reliability of functional protein expression, this approach may represent a paradigm shift for heterologous protein expression having important consequences, both for structural biology and biotechnology. One may anticipate that there are other control mechanisms that affect co-translational folding, including but not limited to the availability of tRNA isoacceptor molecules under different growth conditions. It is thought that codon usage patterns are not only species-specific, but also tissue-specific in eukaryotes, that selective codon usage may reflect an important regulatory mechanism³⁶. The studies presented here underscore the importance of achieving general solutions to problems of heterologous protein expression. Scientific advances based on integration of proteomic and genomic analysis may not be fully realized until the target genes and the synthetic potential of the expression organism are harmonized; failing to achieve such a balance may leave many potential products undiscovered. The value of codon harmonization is that it may provide another strategy for improving the yield and quality of heterologously expressed proteins, particularly in areas of vaccine and biopharmaceutical development.

### MATERIALS AND METHODS

### Algorithm for Codon Harmonization.

Design of synthetic genes with this algorithm requires two major steps: identifying and recoding link/end segments, followed by the re-coding of other areas of the gene. Identifying and recoding a link/end segment is comprised of four steps: a) calculating average codon usage frequencies for consecutive groups of three contiguous codons over the entire gene sequence for the target gene sequence in its native host; b) calculating the threshold value for infrequent codon usage in the native host (defined as the average of the median codon usage rate for the ten amino acids (Tyr, His, Trp, Ile, Leu, Val, Ser, Thr, Pro, and Cys) that appear most frequently in link/end segments of *E*. *coli*; c) identifying segments of the protein, comprised of 15 contiguous amino acids, in which one or more of these "link/end segment associated residues" appears at least two times and their respective codons are used less frequently than the calculated threshold value, and d) replacing link/end segment codons used less frequently than the threshold value with synonymous *E*. *coli* codons having usage frequencies that are less than or equal to those of the specified codons in the native gene. For *P. falciparum,* the threshold usage frequency is 33.6% (Table 1). After identifying and harmonizing the usage frequencies of critical codons in the link/end segments, the rest of the gene is harmonized by selecting synonymous codons from the heterologous expression host having usage frequencies that best reflect the usage frequencies found for the native gene in the native expression host. The selection logic is as follows: preferably equal to, but if not available then the nearest greater than or less than.

### Construction of Expression vector pET(K)

A multi-step cloning strategy was used to generate the final expression vector, pET(K) from a precursor known as pET(AT)³⁷. Elimination of *bla* gene expression from pET(AT) was achieved by excising the transcriptional and translational regulatory region with DraI (New England Biolabs, Beverly, MA) and SspI (Roche, Indianapolis, IN) and re-ligating the ends, producing pET(T), which confers tetracycline resistance. The pET(K) was prepared from linearized pET(T), which was prepared by digesting with Tth111I (New England Biolabs), treating with T4 DNA polymerase (Roche) to generate blunt ends and digesting with SapI (Roche). The *kan* gene was isolated from the pZE2.1PAC.4 vector³⁸ by digesting with AatII (Roche) and SacI (Roche), and a blunt ended insert was prepared by treating with T4 DNA Polymerase (Roche). Restriction mapping ofpET(K) showed that the *kan* coding sequence is coded from the plus strand.

### Design, preparation and cloning of the synthetic gene fragments

### 1. Site-directed mutagenesis of MSP1₄₂ FVO / FMP003.

The codon harmonization algorithm was applied to the gene fragment encoding MSP1₄₂ from the FVO strain of *P. falciparum* (GenBank accession no. L20092). A single synonymous codon change, associated with a predicted N-terminal link/end segment of the protein (codon 141 (Ile); by 423 (C->A), was selected for harmonization. Mutagenesis, cloning and expression of this synthetic gene fragment, defined as FMP003 has been described elsewhere³².

### 2. Full codon harmonization of MSP1₄₂ FVO gene:

The synthetic gene sequence for the MSP1₄₂ FVO was developed by applying the codon harmonization algorithm over the entire gene sequence (Genbank Accession no. DQ926900). For synthesis, consecutive pairs of complementary oligonucleotides (each 50-60 bases, having 12-13 bases of unpaired sequence on the 5' ends) were used to prepare four separate larger segments by using sequential PCR steps. After TA cloning³⁹ and transformation of One Shot TOP 10 supercompetent cells (Invitrogen, Carlsbad, CA) using ampicillin resistance, transformants were isolated and inserts were sequenced to confirm all codon modifications. Each of the four segments of ~ 300 nt contained unique restriction enzyme sites at their termini, which allowed final assembly: fragment 1, 5' NdeI - 3' HincII; fragment 2, 5' HincII - 3' BsrG; fragment 3, 5' BsrG I - 3' BstBI; fragment 4, 5' BstBI - 3'XhoI. The assembled MSP1₄₂ fragment (1130 nt) was cloned into the TA vector pCR 2.1 (Invitrogen), producing pCR 2.1-MSP1₄₂ FVO, and transformants were isolated and verified by DNA sequencing. The MSP1₄₂ FVO insert was excised from pCR 2.1-MSP1₄₂ FVO by digesting with NdeI and XhoI, the insert was purified on a 1 % agarose gel, and cloned into the final pET(K) expression vector also prepared with NdeI and XhoI. Competent host cells (One Shot TOP 10) were transformed to kanamycin resistance, and restriction analysis was used to identify correct clones for DNA sequencing. For expression, the plasmid was used to transform the BL21-DE3 expression host (Novagen, Milwaukee, WI). The final construction is called pET(K) MSP1₄₂ (FVO) or FMP010.

### 3. Full codon harmonization of MSP1₄₂ 3D7.1 gene:

The sequence for the synthetic MSP1₄₂ 3D7.1 gene fragment was also developed by applying the full codon harmonization algorithm (Genbank Accession no. DQ926901). The gene was constructed by using PCR amplification in conjunction with proprietary protocols (Retrogen, Inc., San Diego, CA). The amplified DNA's were inserted into the pCR-Blunt vector (Invitrogen) creating the vector pCR 2.1-MSP142 3D7.1. Both strands of the pCR 2.1-MSP1₄₂ 3D7.1 insert were verified by sequencing using an ABI 377 automated sequencer, and confirmed to be correct. The insert was excised from pCR 2.1-MSP1₄₂ 3D7.1 by digesting with NdeI and NotI, purified on a 1 % agarose gel, and subcloned into the final pET(AT) expression vector, which was also prepared by digesting with NdeI and NotI, producing pET(AT) MSP1₄₂ 3D7.1. BL21-DE3 transformants were selected on ampicillin, and authenticity of the MSP1₄₂ insert within pET(AT) MSP1₄₂ 3D7.1 was verified by sequencing purified plasmid.

### 4. Full codon harmonization of MSP1₄₂ Camp gene:

The Camp allele of MSP1₄₂ is a chimera between the 3D7 allele (1122 bp) and the FVO allele (1070 bp). Thus the codon harmonized Camp gene fragment was created by splicing together by 1-894 from the MSP1₄₂ 3D7.1 and by 842-1070 of the MSP1₄₂ FVO gene fragments described above. Plasmids containing the inserts were digested with AlwNI (New England Biolabs). The pET(AT) MSP1₄₂ 3D7.1 plasmid served as the vector DNA, and provided the 5' end of the gene, while the FMP010 plasmid containing the FVO gene, , provided the 3' end of the gene. The vector fragment was treated with Shrimp Alkaline phosphatase (Roche) to dephosphorylate ends. Vector and insert were gel purified using QIAEX II (QIAGEN, Valencia, CA) and ligated using T4 DNA ligase (Roche). Ligations were used to transform electrocompetent B834 DE3 bacterial cells (Novagen) using tetracycline resistance. An intermediate clone, pET(T) MSP1₄₂ Camp was generated, and it's insert was used for the final construction of the pET(K) MSP1₄₂ Camp expression vector. Briefly, both the insert and pET(K) vector DNA's were prepared by digestion with NdeI and NotI, the digested DNA's were gel purified, ligated and transformed into BL21-DE3. Colonies were screened for correct inserts using DNA restriction digestion and analyzed on ethidium stained 1 % agarose gels. Correct clones were tested for expression using standard expression techniques and were sequenced to confirm correct sequence (Genbank Accession no. DQ926902).

### 5. Codon harmonized LSA-NRCH gene fragment

A codon harmonized synthetic gene was comprised of fragments from the N-terminus (residues 28-154) and the C-terminus (residues 1630-1909) as well as two 17 amino acid repeats of LSA-1 of the *P. falcipcarum* 3D7 clone (Genebank ID no. A45592). The molecular construction of the LSA-NRCH synthetic gene (GenBank Accession ID no. AY751501) and expression from the pET(K) expression vector have been described elsewhere 22.

### 6. Codon optimized LSA-NRCE gene fragment

A synthetic gene encoding the same protein sequence as described above for the LSA-NRC^{H} was designed based on using the most abundant codons associated with highly expressed proteins in *E*. *coli*⁴⁰*.* The gene was synthesized commercially (Retrogen, San Diego, CA), cloned and verified as described for the MSP1₄₂ (3D7) gene fragment above. The synthetic gene was ligated into the NdeI and NotI sites of pET(K) to prepare the expression construct pET(K) LSA-NRC^{E}. The recombinant plasmid was amplified by transforming *E. coli* DH5α using kanamycin resistance, and the gene insert was verified by sequencing both DNA strands. For expression of the LSA- NRC^{E} protein, the plasmid was used to transform *E. coli* Tuner (DE3) (Novagen) using kanamycin resistance in the presence of 1 % glucose.

### Expression

Shake flasks containing 100 mL of phytone-based super broth and 0.5% glucose (all MSP1₄₂ constructions) or Select APS Superbroth medium (Difco, Becton Dickinson, Sparks, MD) and 1% glucose (all LSAT constructions) were inoculated with 0.1 mL of cryopreserved cells and incubated in a shaking incubator at 175 rpm and 30 °C (all MSP1₄₂ constructions) or 37 °C (all LSA-NRC constructions) overnight for 10-14 hours. Cultures also contained the appropriate antibiotic for the clone (100 *µ*g/ml ampicillin for MSP1₄₂ 3D7 and 50 *µ*g/ml kanamycin for MSP1₄₂ Camp and MSP1₄₂ FVO (designated FMP010), and 35 *µ*g/ml kanamycin for all LSA-NRC constructions). The overnight cultures were used to inoculate 10 L fermentors filled with the media and antibiotics described above for each clone, and the cultures were fermented at the appropriate temperatures with either 400 or 600 rpm of agitation to an optical density at 600 nm of 4-7 OD (all MSP1₄₂ constructions) or 8-10 (all LSA-NRC constructions). In all cases, protein expression was induced for 2-3 hours by adding dioxane-free IPTG (Gold Biolabs, St. Louis, MO) to a final concentration of 0.1 mM. Bacteria were harvested by centrifugation at 4 °C, 15,000 rpm and the cell paste was stored frozen at -80 ± 10 °C.

### Evaluation of protein expression and immunoblotting:

Protein expression was evaluated either by staining with Coomassie Blue R250 (Bio-Rad, Richmond, CA) after SDS-PAGE⁴¹ of whole cell extracts, or by western blotting of purified proteins. In either case, proteins were separated under nonreducing conditions. Proteins were electrophoresed with Tris-Glycine buffering (Invitrogen), on 4-20% gradient gels. Whole cell extracts were prepared by extracting 0.5 OD₆₀₀ of cells with SDS-PAGE sample buffer. For protein purifications, frozen cell paste was thawed and cells were lysed by microfluidization (Model M-110Y, Microfluidics Corp. Newton, MA) as described previously³⁷. For western blotting, MSP1₄₂ proteins were detected by probing with polyclonal rabbit anti-MSP-1₄₂³⁷ while LSA-NRC proteins were detected by probing with mouse anti-6xHis antiserum²² (6xHis tag disclosed as SEQ ID NO: 10). Western blots were prepared by using nitrocellulose membranes (PROTRAN, Schleicher & Schuell, Inc., Keene, NH) which were subsequently blocked using 5% nonfat dry milk and 0.1% Tween 20 in PBS, pH 7.4. The secondary antibodies were anti-rabbit IgG (Fc) or anti-mouse IgG (H +L) alkaline phosphatase conjugates (Promega, Madison, WI) and reactions were detected with nitro-blue tetrazolium (NBT) and 5-bromo-4-chloro-3-indolyl phosphate (BCIP) (Sigma Chemicals) in 100mM NaCl, 5mM MgCl₂, 100mM Tris-HCl, pH 9.5. All antibodies were diluted into phosphate buffered saline, pH 7.4 containing 0.1% Tween 20, and the same buffer was used for all washing steps. MSP1₄₂ proteins were further evaluated for maintenance of structure by probing western blots with six different conformation-dependant mAb⁴². These studies required at least partial purification of the expressed proteins by chromatography on Ni²⁺ chelate (Ni-NTA Superflow, QIAGEN, Valencia, CA) in order to allow for epitope stabilization by formation of intra-molecular disulfide bridges within the C-termini of the molecules ^{32; 37}.

### RESULTS

### Codon Harmonization Algorithm

The identification of transcript regions that are likely to promote ribosomal pausing requires a means to predict such "link/end" segments. We decided to evaluate coding sequence for the presence of those amino acids most often associated with such regions, and to apply a standardized computation to determine operationally if such a region contains infrequently used codons. Table 1 shows the calculated threshold value for infrequent codon usage for these residues. The threshold calculation (average of the median usage value for the ten relevant amino acids) was biased toward lower usage frequency by calculating the median usage value for each relevant amino acid rather than its mean usage value. For *P. falciparum* the threshold value was calculated to be 33.6%. A potential link/end segment was calculated to be present if within a 15-residue range two or more codons existed that encoded at least one of the ten relevant amino acids, i.e. those that appear most often in link/end segments, and their usage frequencies were less than 33.6% (averaged from three contiguous codons). Fig. 1 shows an example of a region of the FVO allele of the MSP1₄₂ gene fragment that was calculated to be a link/end segment (top line). Nine of the 20 amino acids shown in this diagram are encoded by isoacceptor codons that are used 20% of the time or less in the native expression host, and among these, four (I141, Y151, L152, P153) belong to the group of ten residues that appear most frequently in the link/end segments of *E*. *coli* proteins. Although I158 satisfies the criterion of being among these ten residues, its isoacceptor usage frequency in the native expression host is greater than 33.6%; therefore this residue is not calculated to be part of the link/end segment. Introduction of the native *P. falciparum* gene sequence into *E*. *coli* results in a dramatic shift in usage frequencies distributed over the length of the sequence (compare first and second lines). Of the nine low frequency codons identified in the first line, five are shifted to higher usage frequencies that exceed the threshold; I141 and Y151 are among these. In addition several codons that are used at high frequency in the native host are used at low frequency in *E*. *coli,* namely Q145, N148, Y154, L155, and N157. The usage frequency for the link/end segment residue P153 (CCC) drops from 11 % in *P. falciparum* to 0.1 % in *E. coli.* Following full codon harmonization of this segment for expression in *E*. *coli* (i.e., matching the frequency of codon usage at each residue), codon usage patterns for heterologous expression are in good agreement with patterns for expression of the native gene in the native host (compare first and third line), with the usage rate for P153 increasing to 8% as compared to 11% in *P. falciparum.* Our initial studies evaluated the utility of this codon harmonization algorithm by focusing on this region of the gene because, of all of the putative link/end segments that were identified, this was the most discordant when its codon usage patterns were compared for native and heterologous expression.

### Single Codon Application of Codon Harmonization

We wanted to test the value of the algorithm by focusing only on relevant residues in the native sequence that should comprise a link/end segment and whose usage frequency during heterologous expression would exceed the threshold value of 33.6%. Thus we determined that mutating codons for 1141 (ATC→ATA) and Y151 (TAC→TAT) would be sufficient to meet this objective (Fig. 1, compare first and fourth line, also shaded boxes on AA residue line). Although we isolated a mutant that contained the 1141 (ATC→ATA) conversion, we did not capture any mutants containing an Y151 (TAC→TAT) conversion (Fig. 1, fifth line). *E. coli* transformed with the gene, containing the single 1141 (ATC→ATA) conversion (designated FMP003) expressed significantly more protein than was obtained from the wild type gene. Although, we were not able to detect expression of the protein from the codon harmonized directly in cell lysates on gels stained with Coomassie Blue (Fig 2A), however it was detected by western blotting while expression of protein from the wild type gene was not (Fig 2B). Expression of FMP003 allowed for isolation of at least 10-fold more protein per gram of wet cells than did expression of the wild type gene sequence (Fig. 2C). The purified FMP003 protein was determined to be soluble based on its presence in the supernatant fraction after centrifugation at 100,000 x g for I h (data not shown).

### Full Gene Codon Harmonization

The algorithm was further tested by preparing a "fully harmonized" MSP1₄₂ (FVO) gene fragment (designated FMP010) that was recoded to enable efficient translational pausing during expression in *E. coli.* Densitometry of the gel shown in Fig. 3 shows that after induction with IPTG, expression levels obtained from the harmonized gene sequence, FMP010, exceeded the wild type sequence by at least 100-fold. Protein in the cell lysate after microfluidization was fully soluble in the presence of 0.3% sarkosyl, remaining in the supernatant fraction after sequentially centrifuging at 30,000 and 100,000 x g for 1 h (Fig. 4, f1 and f3, respectively), and was absent from the corresponding pellets (Fig 4, f2 and f4, respectively.).

Since we observed a significant increase in total expression for the FMP010 protein following application of this algorithm to the full gene, we decided to apply the same method to the expression of MSP1₄₂ (3D7) and MSP1₄₂ (Camp), which are alternative alleles to FVO. Three hours after IPTG induction, expression levels of MSP1₄₂ (3D7) from the codon harmonized gene were significantly higher than those produced from the native sequence (Fig. 5, CH and WT, respectively, lanes 3), giving approximately a four-fold increase in product yield from 0.8 mg/g wet cells for the native sequence to 3.0 mg/g wet cells from the codon harmonized sequence. Basal levels of expression prior to induction are shown in lanes marked U (un-induced cells). The Camp allele of MSP1₄₂ is a chimera of the FVO and 3D7 alleles. The FVO and 3D7 alleles have different protein sequences (58% identity) 21 within the N-terminal 300 residues of the protein, known as MSP1₃₃, but differ only by four single amino acid changes within the mature C-terminal 96 amino acid fragment known as MSP1₁₉ (Q14/ E14, K61/T61, N70/S70, and G71/R71, respectively; numbering relative to the MSP1₁₉ fragment). The Camp allele is identical to the 3D7 allele within the MSP1₃₃ domain and the N-terminal 80% of MSP1₁₉, including E14, but changes to FVO (K61, N70, and G71) for the C-terminal 20% of MSP1₁₉. Expression results for codon harmonized MSP1₄₂ (Camp), at 12.0 mg/g wet cells (Fig 5, Panel B) were dramatic compared with expression of wild type MSP1₄₂ (Camp), where no expression was detected (not shown).

Expression of the codon harmonized LSA-NRC^{H} malaria vaccine candidate, which has been described elsewhere ²², has proven to be the best approach, to date, for the production of this protein in *E*. *coli.* Expression of native *P.falciparum* sequence encoding the individual N-terminal or C-terminal domains of LSA-1 alone, gives poor yields and unstable plasmids. The same is true for the LSA-NRC^{E} gene, a synthetic gene that was "codon optimized" by substituting with codons that are used frequently in *E*. *coli* proteins (Hillier, Ware, et al 2005). For this later construct, plasmid loss was especially problematic during exponential growth phase, suggesting that the expression of the product induced a host cell stress response. On the other hand, the synthetic gene designed by using codon harmonization (LSA-NRC^{H}) yielded a dramatic increase in expression levels when compared with LSA-NRC^{E} (Fig. 6, Lane I). Furthermore, the protein was soluble, even in the absence of sarkosyl, and the problem of plasmid retention during exponential growth was corrected. These results further support the hypothesis that the LSA-NRC^{E} and LSA-NRC^{H} protein products folded differently when expressed in *E*. *coli* and that the expression of LSA-NRC^{H} protein did not induce a deleterious host cell stress response.

Although the foregoing invention has been described in detail for purposes of clarity of understanding, it will be obvious that certain modifications may be practiced within the scope of the appended claims. All publications and patent documents cited herein are hereby incorporated by reference in their entirety for all purposes to the same extent as if each were so individually denoted. Unless otherwise apparent from the context, each element, feature or embodiment of the invention can be used in combination with any other.

### REFERENCES

1. Adzhubei, A. A., Adzhubei, I. A., Krasheninnikov, I. A. & Neidle, S. (1996). Non-random usage of 'degenerate' codons is related to protein three-dimensional structure. FEBS Lett 399, 78-82.
2. Kurland, C. & Gallant, J. (1996). Errors of heterologous protein expression. Curr Opin Biotechnol 7, 489-493.
3. Kleizen, B., van Vlijmen, T., de Jonge, H. R. & Braakman, I. (2005). Folding of CFTR is predominantly cotranslational. Mol Cell 20, 277-287.
4. Kramer, G., Ramachandiran, V. & Hardesty, B. (2001). Cotranslational folding--omnia mea mecum porto? Int J Biochem Cell Biol 33, 541-553.
5. Svetlov, M. S., Kommer, A., Kolb, V. A. & Spirin, A. S. (2006). Effective cotranslational folding of firefly luciferase without chaperones of the Hsp70 family. Protein Sci 15, 242-247.
6. Etchells, S. A. & Hart1, F. U. (2004). The dynamic tunnel. Nat Struct Mol Biol 11, 391-392.
7. Woolhead, C. A., McCormick, P. J. & Johnson, A. E. (2004). Nascent membrane and secretory proteins differ in FRET-detected folding far inside the ribosome and in their exposure to ribosomal proteins. Cell 116, 725-736.
8. Baram, D. & Yonath, A. (2005). From peptide-bond formation to cotranslational folding: dynamic, regulatory and evolutionary aspects. FEBS Lett 579, 948-954.
9. Berisio, R., Schluenzen, F., Harms, J., Bashan, A., Auerbach, T., Baram, D. & Yonath, A. (2003). Structural insight into the role of the ribosomal tunnel in cellular regulation. Nat Struct Biol 10, 366-370.
10. Wolin, S. L. & Walter, P. (1988). Ribosome pausing and stacking during translation of a eukaryotic mRNA. Embo J7, 3559-3569.
11. Varenne, S., Buc, J., Lloubes, R. & Lazdunski, C. (1984). Translation is a non-uniform process. Effect oftRNA availability on the rate of elongation of nascent polypeptide chains. J Mol Biol 180,549-576.
12. Bulmer, M. (1987). Coevolution of codon usage and transfer RNA abundance. Nature 325, 728-730.
13. Gouy, M. & Gautier, C. (1982). Codon usage in bacteria: correlation with gene expressivity. Nucleic Acids Res 10, 7055-7074.
14. Ikemura, T. (1981). Correlation between the abundance of Escherichia coli transfer RNAs and the occurrence of the respective codons in its protein genes: a proposal for a synonymous codon choice that is optimal for the E. coli translational system. J Mol Biol 151, 389-409.
15. Ikemura, T. (1981). Correlation between the abundance of Escherichia coli transfer RNAs and the occurrence of the respective codons in its protein genes. J Mol Biol 146, 1-21.
16. Thanaraj, T. A. & Argos, P. (1996). Ribosome-mediated translational pause and protein domain organization. Protein Sci 5, 1594-1612.
17. Oresic, M. & Shalloway, D. (1998). Specific correlations between relative synonymous codon usage and protein secondary structure. J Mol Biol 281, 31-48.
18. Thanaraj, T. A. & Argos, P. (1996). Protein secondary structural types are differentially coded on messenger RNA. Protein Sci 5, 1973-1983.
19. Phoenix, D. A. & Korotkov, E. (1997). Evidence of rare codon clusters within Escherichia coli coding regions. FEMS Microbiol Lett 155, 63-66.
20. Weber, J. L. (1987). Analysis of sequences from the extremely A + T-rich genome of Plasmodium falciparum. Gene 52, 103-109.
21. Tanabe, K., Mackay, M., Goman, M. & Scaife, J. G. (1987). Allelic dimorphism in a surface antigen gene of the malaria parasite Plasmodium falciparum. J Mol Biol 195, 273-287.
22. Hillier, C. J., Ware, L. A., Barbosa, A., Angov, E., Lyon, J. A., Heppner, D. G. & Lanar, D. E. (2005). Process development and analysis of liver-stage antigen 1, a preerythrocyte-stage protein-based vaccine for Plasmodium falciparum. Infect Immun 73, 2109-2115.
23. Stenstrom, C. M., Holmgren, E. & Isaksson, L. A. (2001). Cooperative effects by the initiation codon and its flanking regions on translation initiation. Gene 273, 259-265.
24. Stenstrom, C. M., Jin, H., Major, L. L., Tate, W. P. & Isaksson, L. A. (2001). Codon bias at the 3'-side of the initiation codon is correlated with translation initiation efficiency in Escherichia coli. Gene 263, 273-284.
25. Chen, G. F. & Inouye, M. (1990). Suppression of the negative effect of minor arginine codons on gene expression; preferential usage of minor codons within the first 25 codons of the Escherichia coli genes. Nucleic Acids Res 18, 1465-1473.
26. Zhang, S., Goldman, E. & Zubay, G. (1994). Clustering of low usage codons and ribosome movement. J Theor Biol 170, 339-354.
27. Frank, J., Verschoor, A., Li, Y., Zhu, J., Lata, R. K., Radermacher, M., Penczek, P., Grassucci, R., Agrawal, R. K. & Srivastava, S. (1995). A model of the translational apparatus based on a three-dimensional reconstruction of the Escherichia coli ribosome. Biochem Cell Biol 73, 757-765.
28. Frank, J., Zhu, J., Penczek, P., Li, Y., Srivastava, S., Verschoor, A., Radermacher, M., Grassucci, R., Lata, R. K. & Agrawal, R. K. (1995). A model of protein synthesis based on cryo-electron microscopy of the E. coli ribosome. Nature 376, 441-444.
29. Deuerling, E., Schulze-Specking, A., Tomoyasu, T., Mogk, A. & Bukau, B. (1999). Trigger factor and DnaK cooperate in folding of newly synthesized proteins. Nature 400, 693-696.
30. Ferbitz, L., Maier, T., Patzelt, H., Bukau, B., Deuerling, E. & Ban, N. (2004). Trigger factor in complex with the ribosome forms a molecular cradle for nascent proteins. Nature 431, 590-596.
31. Netzer, W. J. & Hard, F. U. (1998). Protein folding in the cytosol: chaperonin-dependent and -independent mechanisms. Trends Biochem Sci 23, 68-73.
32. Darko, C. A., Angov, E., Collins, W. E., Bergmann-Leitner, E. S., Girouard, A. S., Hitt, S. L., McBride, J. S., Diggs, C. L., Holder, A. A., Long, C. A., Barnwell, J. W. & Lyon, J. A. (2005). The clinical-grade 42-kilodalton fragment of merozoite surface protein 1 of Plasmodium falciparum strain FVO expressed in Escherichia coli protects Aotus nancymai against challenge with homologous erythrocytic-stage parasites. Infect Immun 73, 287-297.
33. Komar, A. A., Lesnik, T. & Reiss, C. (1999). Synonymous codon substitutions affect ribosome traffic and protein folding during in vitro translation. FEBS Lett 462, 387-391.
34. Pan, W., Ravot, E., Tolle, R., Frank, R., Mosbach, R., Turbachova,I. & Bujard, H. (1999). Vaccine candidate MSP-1 from Plasmodium falciparum: a redesigned 4917 by polynucleotide enables synthesis and isolation of full-length protein from Escherichia coli and mammalian cells. Nucleic Acids Res 27, 1094-1I03.
35. Singh, S., Kennedy, M. C., Long, C. A., Saul, A. J., Miller, L. H. & Stowers, A. W. (2003). Biochemical and immunological characterization of bacterially expressed and refolded Plasmodium falciparum 42-kilodalton C-terminal merozoite surface protein 1. Infect Immun 71, 6766-6774.
36. Plotkin, J. B., Robins, H. & Levine, A. J. (2004). Tissue-specific codon usage and the expression of human genes. Proc Natl Accad Sci U S A 101, 12588-12591.
37. Angov, E., Aufiero, B. M., Turgeon, A. M., Van Handenhove, M., Ockenhouse, C. F., Kester, K. E., Walsh, D. S., McBride, J. S., Dubois, M. C., Cohen, J., Haynes, J. D., Eckels, K. H., Heppner, D. G., Ballou, W. R., Diggs, C. L. & Lyon, J. A. (2003). Development and pre-clinical analysis of a Plasmodium falciparum Merozoite Surface Protein-1(42) malaria vaccine. Mol Biochem Parasitol 128, 195-204.
38. Lutz, R. & Bujard, H. (1997). Independent and tight regulation of transcriptional units in Escherichid coli via the LacR/O, the TetR/O and AraC/I1-I2 regulatory elements. Nucleic Acids Res 25, 1203-1210.
39. Holton, T. A. & Graham, M. W. (1991). A simple and efficient method for direct cloning of PCR products using ddT-tailed vectors. Nucleic Acids Res 19, 1156.
40. Andersson, S. G. & Kurland, C. G. (1990). Codon preferences in free-living microorganisms. Microbiol Rev 54, 198-210.
41. Laemmli, U. K. (1970). Cleavage of structural proteins during the assembly of the head of bacteriophage T4. Nature 227, 680-685.
42. Burghaus, P. A. & Holder, A. A. (1994). Expression of the 19-kilodalton carboxy-terminal fragment of the Plasmodium falciparum merozoite surface protein-1 in Escherichia coli as a correctly folded protein. Mol Biochem Parasitol 64, 165-169.

### Clauses

1. A nucleic acid having a coding region pause-harmonized for expression in a recombinant host, the coding region comprising:
   a) a series of codons encoding a protein, or fragment thereof, having an amino acid sequence at least 85% identical to a native protein or fragment thereof, of a native host;
   b) the series of codons encoding a peptide at least 17 amino acids in length; and,
   c) a nucleotide sequence at least 65% identical to a native contiguous codon series encoding the native protein of the native host
      wherein the coding region is formed by substituting at least two infrequently used codons of the native contiguous codon series with synonymous codons, each synonymous codon having a recombinant host codon usage frequency that is less than or equal to the native host codon usage frequency of the substituted infrequently used codons, the substituted infrequently used codons being present in a contiguous series of 15 codons, and expression of the nucleic acid in the recombinant host provides at least about 10% increased yield of the protein.
2. The nucleic acid of clause 1 , wherein the infrequently used codons encode for an amino acyl residue selected from the group consisting of Cys, His, Ile, Leu, Pro, Ser, Thr, Trp, Tyr and Val.
3. The nucleic acid of clause 1, wherein the protein of a) is 95% identical to a native protein or fragment thereof, of a native host.
4. The nucleic acid of clause 3, wherein the protein of a) is identical to a native protein or fragment thereof, of a native host.
5. The nucleic acid of clause 1, wherein the series of codons encodes a peptide at least 25 amino acids in length.
6. The nucleic acid of clause 5, wherein the series of codons encodes a peptide at least 50 amino acids in length.
7. The nucleic acid of clause 1, wherein the substituted infrequently used codons are present in a contiguous series of 11 codons.
8. The nucleic acid of clause 7, wherein the substituted infrequently used codons are present in a contiguous series of 10 codons.
9. The nucleic acid of clause 1, wherein the recombinant host is a prokaryotic cell.
10. The nucleic acid of clause 1, wherein the native host is a cell derived from a multicellular organism.
11. The nucleic acid of clause 1 , wherein the native protein is a parasite coat protein.
12. The nucleic acid of clause 1 , wherein the native protein is a receptor.
13. The nucleic acid of clause 1, wherein the native protein is an enzyme.
14. An isolated protein, comprising at least 7 amino acyl residues, expressed in a recombinant host and encoded by a coding sequence comprising any 21 or more contiguous nucleic acid residues taken from a nucleic acid sequence (or fragment thereof) selected from the group consisting of SEQ ID NOS: 1, 3, 5, 6 and 8.
15. An isolated protein comprising an amino acyl sequence of at least 7 contiguous amino acyl residues taken from an amino acid sequence selected from the group consisting of SEQ ID NOS: 2, 4, 7 and 9, or any fragment of at least 7 contiguous amino acyl residues therefrom.

## Claims

1. A nucleic acid having a coding region pause-harmonized for expression in a recombinant *E. coli* host, the coding region comprising:
a) a series of codons encoding MSP1₄₂ (FVO), or a fragment thereof, or MSP1₄₂ (Camp) or a fragment thereof, or a variant of MSP1₄₂ (FVO) or a fragment thereof, or a variant of MSP1₄₂ (Camp) or a fragment thereof, having an amino acid sequence at least 85% identical to the native protein or fragment thereof, of the native host *P. falciparum*;
b) the series of codons encoding a peptide at least 17 amino acids in length; and
c) the nucleotide sequence of the series of codons being at least 65% identical to a native contiguous codon series encoding the native MSP1₄₂ (FVO) protein or fragment thereof of the native host *P. falciparum,* or the native MSP1₄₂ (Camp) protein or fragment thereof of the native host *P. falciparum,*
wherein the coding region is formed by substituting at least one infrequently used codon of a link/end (interdomain) segment of the native *P. falciparum* contiguous codon series with a synonymous codon, each synonymous codon having a recombinant *E*. *coli* host codon usage frequency that is less than or equal to the native *P. falciparum* host codon usage frequency of the at least one substituted infrequently used codon, the at least one substituted infrequently used codon being present in a contiguous series of 15 codons, and expression of the nucleic acid in the recombinant *E. coli* host provides at least about 10% increased yield of the protein.

2. The nucleic acid of claim 1, wherein the coding region comprises a series of codons encoding MSP1₄₂ (FVO) or a fragment thereof, or a variant of MSP1₄₂ (FVO) or a fragment thereof, and the nucleotide sequence of the series of codons are at least 65% identical to a native contiguous codon series encoding the native MSP1₄₂ (FVO) protein or fragment thereof of the native host *P. faciparum.*

3. The nucleic acid of claim 1, wherein the coding region comprises a series of codons encoding MSP1₄₂ (Camp) or a fragment thereof, or a variant of MSP1₄₂ (Camp) or a fragment thereof, and the nucleotide sequence of the series of codons are at least 65% identical to a native contiguous codon series encoding the native MSP1₄₂ (Camp) protein or fragment thereof of the native host *P. faciparum.*

4. The nucleic acid of claim 1 , wherein the at least one infrequently used codon encodes for an amino acid residue selected from the group consisting of Cys, His, Ile, Leu, Pro, Ser, Thr, Trp, Tyr and Val.

5. The nucleic acid of claim 1, wherein the protein variant of a) is 95% identical to the native protein or fragment thereof, of a native host *P. flaciparum.*

6. The nucleic acid of claim 1, wherein the series of codons encodes a peptide at least 25 amino acids in length.

7. The nucleic acid of claim 6, wherein the series of codons encodes a peptide at least 50 amino acids in length.

8. The nucleic acid of claim 1, wherein the coding region is formed by substituting at least two infrequently used codons of a link/end (interdomain) segment of the native *P. falciparum* contiguous codon series with synonymous codons, each synonymous codon having a recombinant *E. coli* host codon usage frequency that is less than or equal to the native *P. falciparum* host codon usage frequency of the substituted infrequently used codons.
